(19) **Europäisches Patentamt European Patent Office Office européen des brevets**

(11) **EP 3 603 527 B1**

(12) **EUROPEAN PATENT SPECIFICATION**

(45) Date of publication and mention of the grant of the patent:
**11.05.2022 Bulletin 2022/19**

(21) Application number: **18775258.9**

(22) Date of filing: **28.03.2018**

(51) International Patent Classification (IPC):
**A61B 8/08** *(2006.01)*    **A61B 8/00** *(2006.01)*

(52) Cooperative Patent Classification (CPC):
**A61B 8/485; A61B 8/5269; A61B 8/54;**
A61B 8/5207; A61B 8/5223

(86) International application number:
**PCT/KR2018/003654**

(87) International publication number:
**WO 2018/182308 (04.10.2018 Gazette 2018/40)**

(54) **ULTRASONIC DIAGNOSTIC DEVICE AND OPERATING METHOD THEREOF**

ULTRASCHALLDIAGNOSEVORRICHTUNG UND BETRIEBSVERFAHREN DAFÜR

DISPOSITIF DE DIAGNOSTIC ULTRASONORE ET PROCÉDÉ DE FONCTIONNEMENT S'Y RAPPORTANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2017 KR 20170039304**

(43) Date of publication of application:
**05.02.2020 Bulletin 2020/06**

(73) Proprietor: **Samsung Medison Co., Ltd.**
**Hongcheon-gun, Gangwon-do 25108 (KR)**

(72) Inventors:
• **KONG, Dong-geon**
**Hwaseong-si**
**Gyeonggi-do 18431 (KR)**
• **LEE, Hyoung-ki**
**Seongnam-si**
**Gyeonggi-do 13527 (KR)**
• **KIM, Hyo-geun**
**Seoul 06354 (KR)**

(74) Representative: **Hylarides, Paul Jacques et al**
**Arnold & Siedsma**
**Bezuidenhoutseweg 57**
**2594 AC Den Haag (NL)**

(56) References cited:
JP-A- 2014 000 260       KR-A- 20110 127 618
KR-A- 20140 045 189      KR-A- 20150 014 315
KR-A- 20150 130 093      US-A1- 2015 173 718

• **COREY F SCOTT ET AL: "Sonic Estimation of ElasticityviaResonance: A New Method of Assessing Hemostasis", ANNALS OF BIOMEDICAL ENGINEERING, SPRINGER US, NEW YORK, vol. 44, no. 5, 23 September 2015 (2015-09-23), pages 1405-1424, XP035897938, ISSN: 0090-6964, DOI: 10.1007/S10439-015-1460-Y [retrieved on 2015-09-23]**
• **DENG YUFENG ET AL: "Ultrasonic Shear Wave Elasticity Imaging Sequencing and Data Processing Using a Verasonics Research Scanner", IEEE TRANSACTIONS ON ULTRASONICS, FERROELECTRICS AND FREQUENCY CONTROL, IEEE, US , vol. 64, no. 1 12 January 2017 (2017-01-12), pages 164-176, XP011638783, ISSN: 0885-3010, DOI: 10.1109/TUFFC.2016.2614944 Retrieved from the Internet: URL:http://ieeexplore.ieee.org/document/75 81103/ [retrieved on 2016-10-03]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 603 527 B1

## Description

TECHNICAL FIELD

The present disclosure relates to an ultrasound diagnostic apparatus, a method of controlling the ultrasound diagnostic apparatus, and a computer program product having stored therein program instructions for performing the method of controlling the ultrasound diagnostic apparatus.

BACKGROUND ART

Recently, in the medical field, various types of medical imaging apparatuses have been widely used to visualize and acquire information about living tissue of a human body for early diagnosis or surgery with regard to various diseases. Representative examples of these medical imaging apparatuses may include an ultrasound diagnostic apparatus, a computed tomography (CT) apparatus, and a magnetic resonance imaging (MRI) apparatus.

Ultrasound diagnostic apparatuses transmit ultrasound signals generated by transducers of a probe to an object and receive information of echo signals reflected from the object, thereby obtaining an image of an internal part of the object. In particular, ultrasound diagnostic apparatuses are used for medical purposes including observing an internal area of an object, detecting foreign substances, and assessing injuries. Such ultrasound diagnostic apparatuses exhibit high stability, display images in real-time, and are safe due to lack of radiation exposure compared to diagnostic X-ray apparatuses. Therefore, ultrasound diagnostic apparatuses have been widely used together with other types of imaging diagnostic apparatuses.

In addition, an ultrasound diagnostic apparatus may provide a brightness (B) mode image representing a reflection coefficient of an ultrasound signal reflected from an object as a two-dimensional (2D) image, a Doppler (D) mode image showing an image of a moving object (in particular, blood flow) by using a Doppler effect, an elastic mode image visualizing a difference between responses when compression is or is not applied to an object, as an image.

In the publication "Sonic Estimation of Elasticity via Resonance: A New Method of Assessing Homostasis", Corey et al, Annals of Biomedical Engineering, Springer US, 44(4), 2015, shear wave imaging is discussed with an emphasis to detect and minimise reverberation.

DESCRIPTION OF EMBODIMENTS

SOLUTION TO PROBLEM

Provided are a method and an ultrasound diagnostic device for processing shear wave elastography data, as well as a computer program product with instructions for performing the method.

ADVANTAGEOUS EFFECTS OF DISCLOSURE

According to the embodiments of the disclosure, it is possible to detect the occurrence of a reverberation and display and notify information about the detected reverberation to a user, thereby improving the accuracy of measurement of elasticity.

Furthermore, when a reverberation occurs, it is possible to allow the user to manipulate a probe such that a severe reverberation may not occur or to move a region of interest (ROI) to a region of mild reverberation, thereby facilitating elasticity measurement and increasing user convenience.

BRIEF DESCRIPTION OF DRAWINGS

The disclosure will now be described more fully through the detailed descriptions below with reference to the accompanying drawings, in which reference numerals denote structural elements.

FIG. 1 is a block diagram of a configuration of an ultrasound diagnostic apparatus according to an embodiment of the disclosure.
FIGS. 2A through 2C illustrate ultrasound diagnostic apparatuses according to an embodiment.
FIG. 3 is a block diagram illustrating components of an ultrasound diagnostic apparatus according to an embodiment of the disclosure.
FIG. 4 is a flowchart of a method, performed by an ultrasound diagnostic apparatus, of detecting the occurrence of a reverberation in a region of interest (ROI) and displaying information about the reverberation, according to an embodiment of the disclosure.
FIG. 5 is a diagram for explaining a method, performed by an ultrasound diagnostic apparatus, of inducing a displacement in a tissue of an ROI and calculating the displacement, according to an embodiment of the disclosure.
FIG. 6 is a flowchart of a method, performed by an ultrasound diagnostic apparatus, of measuring a shear wave arrival time from a tissue displacement in an ROI, according to an embodiment of the disclosure.
FIG. 7A is a graphical representation of coordinates for locations of a plurality of scan lines in an ROI and a focusing direction of a focused ultrasound beam applied to the ROI in a depth direction.
FIG. 7B is a diagram for explaining a method, performed by an ultrasound diagnostic apparatus, of calculating a shear wave propagation velocity at a plurality of measurement points within an ROI, according to an embodiment of the disclosure.
FIG. 7C is a graph illustrating a relationship between a shear wave arrival time and a tissue displacement measured by an ultrasound diagnostic apparatus at each of a plurality of measurement points, according to an embodiment of the disclosure.

FIG. 8 is a flowchart of a method, performed by an ultrasound diagnostic apparatus, of detecting the occurrence of a reverberation based on shear wave arrival times at a plurality of measurement points within an ROI, according to an embodiment of the disclosure.

FIG. 9 is a graph illustrating shear wave arrival times respectively measured by an ultrasound diagnostic apparatus at a plurality of measurement points, according to an embodiment of the disclosure.

FIG. 10 is a flowchart of a method, performed by an ultrasound diagnostic apparatus, of detecting the occurrence of a reverberation based on a shear wave velocity calculated at a plurality of measurement points within an ROI, according to an embodiment of the disclosure.

FIGS. 11A and 11B are graphs for explaining a method, performed by an ultrasound diagnostic apparatus, of determining a value of a reliability measurement index based on a shear wave velocity ratio, according to an embodiment of the disclosure.

FIGS. 12A and 12B are diagrams for explaining a method, performed by an ultrasound diagnostic apparatus, of displaying information about a detected reverberation, according to an embodiment of the disclosure.

BEST MODE

According to an aspect of the present disclosure, a method of processing shear wave elastography data according to claim 1 is proposed.

Before the emitting of the focused ultrasound beam, the method may further include transmitting a first ultrasound signal to the object and generating a reference ultrasound image by using an ultrasound echo signal reflected from the object, and the obtaining of the ultrasound may include transmitting a second ultrasound signal to the object in which the shear wave is induced and respectively obtaining a plurality of shear wave images at the plurality of time points by using an ultrasound echo signal reflected from the object.

The measuring of the shear wave arrival times may include: calculating a plurality of time points when displacements of tissues of the object respectively positioned at the plurality of measurement points respectively reach maximum values by comparing each of the plurality of shear wave images with the reference ultrasound image; and respectively determining the calculated plurality of time points as the shear wave arrival times at the plurality of measurement time points.

The detecting of the reverberation may include: determining an average of the shear wave arrival times respectively corresponding to the plurality of measurement points as a first average shear wave arrival time; determining an average of differences as a second average shear wave arrival time, each difference being between shear wave arrival times at two adjacent points among the plurality of measurement points; and detecting occurrence of the reverberation by comparing a value obtained by dividing the first average shear wave arrival time by the second average shear wave arrival time with a preset threshold.

The detecting of the reverberation may further include detecting the occurrence of the reverberation by comparing the first average shear wave arrival time with a preset reference time.

The detecting of the reverberation includes calculating a first shear wave velocity by dividing an average of distances of the plurality of measurement points by an average of the shear wave arrival times respectively measured at the plurality of measurement points; calculating a second shear wave velocity by dividing a distance between two adjacent points among the plurality of measurement points by a difference between shear wave arrival times respectively measured at the two adjacent measurement points; and detecting occurrence of the reverberation based on the first and second shear wave velocities.

The detecting of the reverberation further includes detecting the occurrence of the reverberation by comparing a difference between the second and first shear wave velocities with a preset threshold.

The detecting of the reverberation may further include determining a value obtained by dividing the difference between the second and first shear wave velocities by the first shear wave velocity as a reliability measurement index (RMI), and the displaying of the information about the detected reverberation comprises displaying the RMI on a display of the ultrasound diagnostic apparatus.

The displaying of the information about the detected reverberation may include displaying the information about the detected reverberation via a user interface including at least one of a phrase, a sentence, a symbol, and a color.

The displaying of the information of the detected reverberation may further include outputting the information about the detected reverberation in the form of a sound including at least one of a beep sound, a melody, and a voice.

According to another aspect of the present disclosure, an ultrasound diagnostic apparatus for processing shear wave elastography data according to claim 5 is proposed.

The ultrasound probe may transmit a first ultrasound signal to the object before emitting the focused ultrasound beam, and the processor may generate a reference ultrasound image by using an ultrasound echo signal reflected from the object, wherein the ultrasound images are a plurality of shear wave images respectively obtained by the processor at the plurality of time points.

The processor may calculate a plurality of time points when displacements of tissues of the object respectively positioned at the plurality of measurement points respectively reach maximum values by comparing each of the plurality of shear wave images with the reference ultrasound image and determine the calculated plurality of time points as the shear wave arrival times at the plurality of measurement time points.

The processor may determine an average of the shear wave arrival times respectively corresponding to the plurality of measurement points as a first average shear wave arrival time, determine an average of differences as a second average shear wave arrival time, each difference being between shear wave arrival times at two adjacent points among the plurality of measurement points, and detect occurrence of the reverberation by comparing a value obtained by dividing the first average shear wave arrival time by the second average shear wave arrival time with a preset threshold.

The processor may detect the occurrence of the reverberation by comparing the first average shear wave arrival time with a preset reference time.

The processor calculates a first shear wave velocity by dividing an average of distances of the plurality of measurement points by an average of the shear wave arrival times respectively measured at the plurality of measurement points, calculate a second shear wave velocity by dividing a distance between two adjacent points among the plurality of measurement points by a difference between shear wave arrival times respectively measured at the two adjacent measurement points, and detect occurrence of the reverberation based on the first and second shear wave velocities.

The processor detects the occurrence of the reverberation by comparing a difference between the second and first shear wave velocities with a preset threshold.

The processor may determine a value obtained by dividing the difference between the second and first shear wave velocities by the first shear wave velocity as an RMI, and the display may display the RMI.

The display may display the information about the detected reverberation via a user interface including at least one of a phrase, a sentence, a symbol, and a color.

According to another aspect of the present disclosure, a computer program product includes a computer-readable storage medium for performing the method of processing shear wave elastography data.

MODE OF DISCLOSURE

The present specification describes principles of the disclosure and sets forth embodiments thereof to clarify the scope of the disclosure and to allow those of ordinary skill in the art to implement the embodiments of the disclosure. The embodiments of the disclosure may have different forms.

Like reference numerals refer to like elements throughout. The present specification does not describe all components in the embodiments of the disclosure, and common knowledge in the art or the same descriptions of the embodiments will be omitted below. The term "module" or "unit" used herein may be implemented as software, hardware, firmware, or any combination of two or more thereof, and according to embodiments, a plurality of "modules" or "units" may be formed as a single element, or one "module" or "unit" may include a plurality of elements.

Hereinafter, the operating principles and embodiments of the disclosure will be described in detail with reference to the accompanying drawings.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

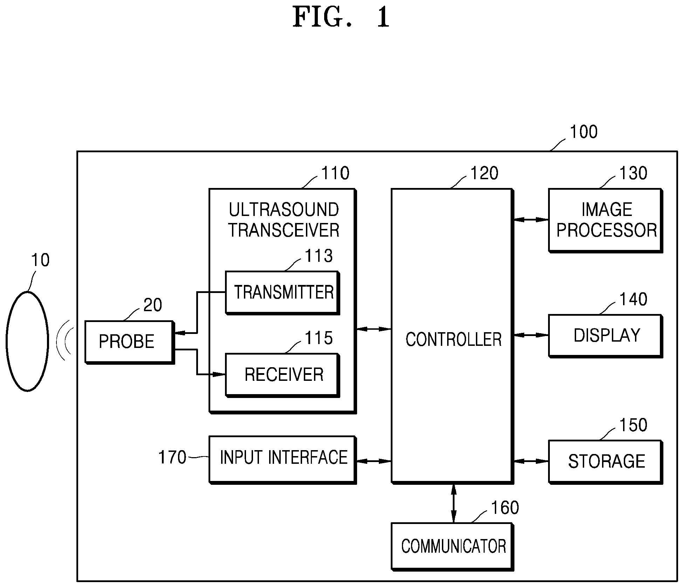

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170. The ultrasound diagnosis apparatus 100 may be of a cart-type or a portable-type ultrasound diagnosis apparatus, which is portable, moveable, mobile, or hand-held.

Examples of the portable-type ultrasound diagnosis apparatus may include a smart phone, a laptop computer, a personal digital assistant (PDA), and a tablet personal computer (PC), each of which may include a probe and a software application, but embodiments are not limited thereto. The probe 20 may include a plurality of transducers.

The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments. The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100.

The ultrasound diagnosis apparatus 100 may include two or more displays 140 according to the present exemplary embodiment. The display 140 may include a touch screen in combination with a touch panel. The controller 120 may control the operations of the ultrasound diagnosis apparatus 100 and flow of signals between the internal elements of the ultrasound diagnosis apparatus 100.

The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus. The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses.

For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160. A program for controlling the ultrasound diagnosis apparatus 100 may be installed in the external apparatus.

The program may include command languages to perform part of operation of the controller 120 or the entire operation of the controller 120.

The program may be pre-installed in the external apparatus or may be installed by a user of the external apparatus by downloading the program from a server that provides applications.

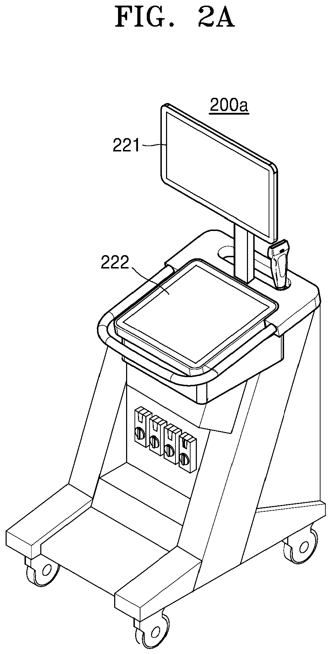

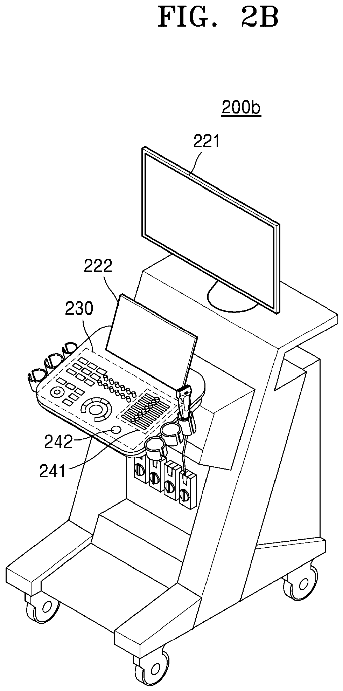

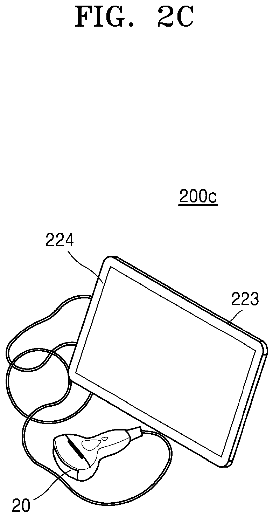

The server that provides applications may include a recording medium where the program is stored. The storage 150 may store various data or programs for driving and controlling the ultrasound diagnosis apparatus 100, input and/or output ultrasound data, ultrasound images, applications, etc. The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto. An example of the ultrasound diagnosis apparatus 100 according to the present exemplary embodiment is described below with reference to FIGS. 2A, 2B, and 2C. FIGS. 2A, 2B, and 2C are diagrams illustrating ultrasound diagnosis apparatus according to an exemplary embodiment. Referring to FIGS. 2A and 2B, the ultrasound diagnosis apparatuses 200a and 200b may include a main display 221 and a sub-display 222. At least one among the main display 221 and the sub-display 222 may include a touch screen.

The main display 221 and the sub-display 222 may display ultrasound images and/or various information processed by the ultrasound diagnosis apparatus 200a or 200b. The main display 221 and the sub-display 222 may provide graphical user interfaces (GUI), thereby receiving user's inputs of data to control the ultrasound diagnosis apparatus 200a or 200b. For example, the main display 221 may display an ultrasound image and the sub-display 222 may display a control panel to control display of the ultrasound image as a GUI. The sub-display 222 may receive an input of data to control the display of an image through the control panel displayed as a GUI. The ultrasound diagnosis apparatus 200a or 200b may control the display of the ultrasound image on the main display 221 by using the input control data.

Referring to FIG. 2B, the ultrasound diagnosis apparatus 200b may include a control panel 230. The control panel 230 may include buttons, trackballs, jog switches, or knobs, and may receive data to control the ultrasound diagnosis apparatus 200b from the user.

For example, the control panel 230 may include a time gain compensation (TGC) button 241 and a freeze button 242. The TGC button 241 is to set a TGC value for each depth of an ultrasound image. Also, when an input of the freeze button 242 is detected during scanning an ultrasound image, the ultrasound diagnosis apparatus 200b may keep displaying a frame image at that time point.

The buttons, trackballs, jog switches, and knobs included in the control panel 230 may be provided as a GUI to the main display 221 or the sub-display 222. Referring to FIG. 2C, the ultrasound diagnosis apparatus 200c may include a portable device. An example of the portable ultrasound diagnosis apparatus 200c may include, for example, smart phones including probes and applications, laptop computers, personal digital assistants (PDAs), or tablet PCs, but an exemplary embodiment is not limited thereto. The ultrasound diagnosis apparatus 200c may include the probe 20 and a main body 223. The probe 20 may be connected to one side of the main body 223 by wire or wirelessly. The main body 223 may include a touch screen 224. The touch screen 224 may display an ultrasound image, various pieces of information processed by the ultrasound diagnosis apparatus 200c, and a GUI.

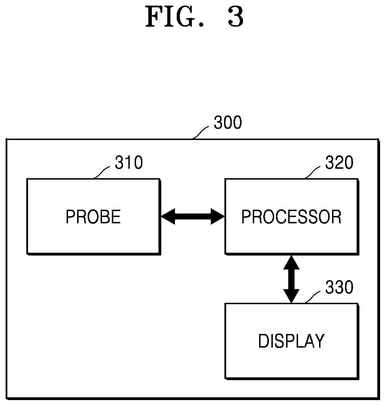

FIG. 3 is a block diagram illustrating components of an ultrasound diagnostic apparatus 300 according to an embodiment of the disclosure.

Referring to FIG. 3, the ultrasound diagnostic apparatus 300 may include a probe 310, a processor 320, and a display 330. According to an embodiment, the ultrasound diagnostic apparatus 300 may include the probe 310 and the processor 320 except for the display 330. Furthermore, according to another embodiment, the ultrasound diagnostic apparatus 300 may further include components other than those shown in FIG. 1

The probe 310 transmits an ultrasound wave to a region of interest (ROI) of an object and detects an echo signal. Furthermore, the probe 310 induces a displacement in the ROI. In an embodiment of the disclosure, the probe 310 may emit a focused beam onto the object to induce a displacement in tissue of the object. The probe 310 may control an ultrasound signal output sequence from piezoelectric elements arranged in an array to generate and output a focused ultrasound beam. When a focused beam is emitted onto the object, the focused beam causes a distortion according to movement of tissue in an axial direction to induce a displacement of the tissue. The probe 310 may propagate a shear wave due to the displacement of tissue in the object. The ultrasound diagnostic apparatus 300 may obtain an elastic mode ultrasound image by scanning an ultrasound image when the displacement is induced in the object.

The processor 320 controls all operations of the ultrasound diagnostic apparatus 300 and processes data and signals. The processor 320 may be composed of one or more hardware units. In an embodiment, the processor 320 may be composed of a hardware unit including a memory for storing at least one of a computer program, an algorithm, and application data and a processor for processing the program, algorithm, or application data stored in the memory. For example, the processor 320 may be composed of a processor including at least one of a central processing unit (CPU), a microprocessor, and a graphic processing unit. In this case, the memory and the processor may be formed as a single chip, but are not limited thereto. According to another embodiment, the processor 320 may be implemented as one or more software modules generated by executing a program code stored in the memory.

According to an embodiment, the processor 320 may include a separate hardware unit that functions as both an image processor and a controller. In this case, the processor 320 may correspond to at least one or a combination of the controller 120 and the image processor 130 of FIG. 1.

The processor 320 calculates a movement displacement of tissue in the ROI from an obtained ultrasound image. For example, a displacement may be calculated by comparing a plurality of ultrasound images obtained before and after applying compression to the object. According to an embodiment, the probe 310 may transmit a first ultrasound signal to an object, and the processor 320 may obtain a reference ultrasound image by using a first ultrasound echo signal reflected from the object. After a focused ultrasound beam is emitted onto the object, the probe 310 may transmit a second ultrasound signal to the object, and the processor 320 may respectively obtain a plurality of shear wave images captured at a plurality of time points based on a second ultrasound echo signal reflected from the object. For example, the second ultrasound signal may be a plane wave.

The processor may calculate displacements of sub-tissues in the object, respectively corresponding to a plurality of measurement points, by comparing each of a plurality of shear wave images with a reference ultrasound image. In an embodiment, the processor 320 may calculate a displacement of a sub-tissue in the object by performing auto-correlation or cross-correlation between the reference ultrasound image and each of the shear wave images. According to another embodiment, a displacement may be calculated by using a differential image between ultrasound images obtained before and after movement of the object, i.e., between a shear wave image and a reference ultrasound image or by differentiating an obtained shear wave image with respect to time. According to an embodiment, the processor 320 may include a module such as a displacement calculator.

The processor 320 may respectively measure shear wave arrival times at a plurality of measurement points that are respectively separated by preset distances from a focal point to which a focused beam is emitted. In an embodiment, the processor 320 may measure shear wave arrival times from displacements of a plurality of sub-tissues in the ROI, and in particular, determine the time when the magnitude of a change in a displacement of a sub-tissue is maximum as a shear wave arrival time. The processor 320 may calculate a time point when a displacement of a tissue in the object positioned at each of a plurality of measurement points is maximum, the measurement points being separated by preset distances from a focal point, and determine the calculated time point as a shear wave arrival time for each of the plurality of measurement points. In this case, the processor 320 may differentiate a plurality of detected tissue displacements with respect to time, respectively calculates axial velocities with respect to time for the differentiated tissue displacements, and respectively determine time points when the calculated axial velocities reach their maximum values as shear wave arrival times at the plurality of measurement points.

According to another embodiment, the processor 320 may measure a shear wave arrival time by calculating, via cross-correlation, a time delay between a displacement signal according to the changes over time at one of the plurality of measurement points and a displacement signal at another measurement point that is adjacent thereto.

The processor 320 may detect a reverberation in an ROI based on the detected tissue displacements and the measured shear wave arrival time. According to an embodiment, the processor 320 may determine an average of shear wave arrival times respectively measured at the plurality of measurement points as a first average shear wave arrival time, determine an average of differences as a second average shear wave arrival time, each difference being between shear wave arrival times at two adjacent points among the plurality of measurement points, and detect the occurrence of a reverberation by comparing a preset threshold with a value obtained by dividing the first average shear wave arrival time by the second average shear wave arrival time. In this case, the processor 320 may detect the occurrence of a reverberation when the first average shear wave arrival time is greater than a preset reference time.

According to an embodiment, the processor 320 may calculate a first shear wave velocity by dividing an average of distances of the plurality of measurement points by an average of shear wave arrival times respectively measured at the plurality of measurement points, calculate a second shear wave velocity by dividing a distance between two adjacent points among the plurality of measurement points by a difference between shear wave arrival times respectively measured at the two adjacent measurement points , and detect the occurrence of a reverberation based on the first and second shear wave velocities. In this case, the processor 320 may calculate a shear wave velocity ratio by dividing a difference between the first and second shear wave velocities by the first shear wave velocity and detect the occurrence of a reverberation by comparing the calculated shear wave velocity ratio with a preset threshold. For example, when the shear wave velocity ratio is greater than or equal to 0.5, the processor 320 may determine that the reverberation has occurred.

According to an embodiment, the processor 320 may calculate a shear wave velocity ratio and obtain a reliability measurement index based on the calculated shear wave velocity ratio.

The display 330 may display an operating state of the ultrasound diagnostic apparatus 300, an ultrasound image, a UI, etc. For example, the display 330 may be constituted by a physical device including at least one of a cathode ray tube (CRT) display, a liquid crystal display (LCD), a plasma display panel (PDP) display, an organic light-emitting display (OLED), a field emission display (FED), a light-emitting diode (LED) display, a vacuum fluorescent display (VFD), a digital light processing (DLP) display, a flat panel display (FPD), a three-dimensional (3D) display, and a transparent display, but is not limited thereto. According to an embodiment, the display 330 may be formed as a touch screen including a touch interface. When the display 330 is formed as a touch screen, the display 330 may be integrated with a user input interface.

The display 330 may display information about a reverberation detected by the processor 320. According to an embodiment, the display 330 may display information about a detected reverberation via a UI including at least one of a phrase, a sentence, a symbol, and a color.

According to an embodiment, the display 330 may display information about a detected reverberation, together with an ultrasound image of an object. In this case, the ultrasound diagnostic apparatus 300 may operate in an elastic mode, and an ultrasound image may be an elastic mode ultrasound image. For example, the information about the detected reverberation may include a RMI.

According to an embodiment, the ultrasound diagnostic apparatus 300 may further include as its component a speaker that outputs information about a detected reverberation in the form of a sound including at least one of a beep sound, a melody, and a voice.

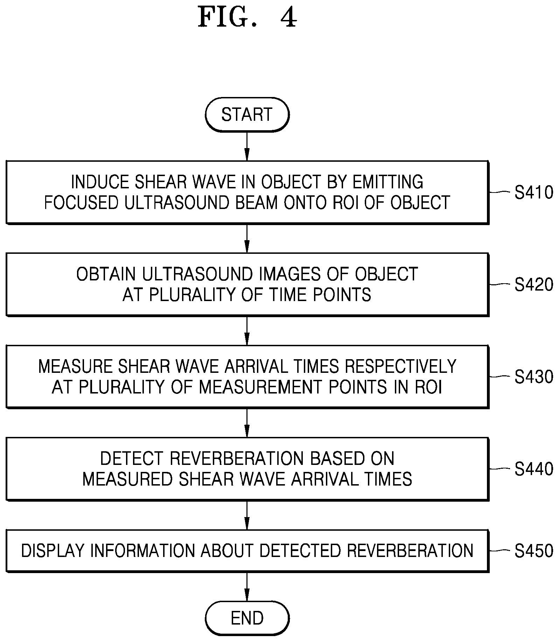

Reference numeral 300 is hereinafter used to collectively denote ultrasound diagnostic apparatuses according to embodiments of the disclosure. However, although reference numerals such as 100, 200a, 200b, and 200c are used to represent the ultrasound diagnostic apparatuses according to embodiments related to specific figures, other embodiments are not excluded, and it will be understood by those of ordinary skill in the art that features of an embodiment may also be applied to other embodiments to which the features are applicable. A method of operating the ultrasound diagnostic apparatus 300 will now be described with reference to FIG. 4.

FIG. 4 is a flowchart of a method, performed by the ultrasound diagnostic apparatus 300, of detecting the occurrence of a reverberation in an ROI and displaying information about the reverberation, according to an embodiment of the disclosure.

In operation S410, the ultrasound diagnostic apparatus 300 induces a shear wave in an object by emitting a focused ultrasound beam onto an ROI of the object. Referring to FIGS. 3 and 4, the probe 310 may induce a displacement in tissue of the object by emitting a focused ultrasound beam onto the ROI of the object. In this case, the focused ultrasound beam may include a pushing pulse. A region of the ROI irradiated with a focused ultrasound beam is referred to as a focal point.

In operation S420, the ultrasound diagnostic apparatus 300 obtains ultrasound images of the object at a plurality of time points. When a focused ultrasound beam is emitted onto the object via the probe 310, a displacement of tissue of the object is induced at a focal point where the focused ultrasound beam is focused. The focused ultrasound beam travels in a depth direction, and the shear wave propagates in a direction that is perpendicular to the displacement and in an axial direction, i.e., from a point where the displacement occurs to both sides along an axis. Subsequently, the probe 310 transmits an ultrasound signal such as a plane wave to the object, and the processor 320 obtains shear wave images captured at a plurality of time points by using an ultrasound echo signal reflected from the object. For example, a shear wave image may be obtained at a frame rate of several thousand frames per second (fps) above 5,000 fps.

In operation S430, the ultrasound diagnostic apparatus 300 respectively measures shear wave arrival times at a plurality of measurement points within the ROI. According to an embodiment, the ultrasound diagnostic apparatus 300 may measure a time point when the magnitude of a change in each of a plurality of tissue displacements is maximum, the tissue displacements respectively corresponding to the plurality of measurement points that are at preset distances away from a focal point, and determine the time point as a shear wave arrival time. Referring to the description with respect to FIG. 3, the processor 320 may calculate a displacement due to movement of a sub-tissue in the object by comparing each of a plurality of shear wave images with a reference ultrasound image.

According to an embodiment, the ultrasound diagnostic apparatus 300 may differentiate tissue displacements in a plurality of shear wave images with respect to time, respectively calculates axial velocities with respect to time for the differentiated tissue displacements, and respectively determine time points when the calculated axial velocities are maximum as shear wave arrival times at the plurality of measurement points.

In operation S440, the ultrasound diagnostic apparatus 300 detects a reverberation based on the measured shear wave arrival time. According to an embodiment, the ultrasound diagnostic apparatus 300 may determine an average of shear wave arrival times respectively measured at the plurality of measurement points as a first average shear wave arrival time, determine an average of differences as a second average shear wave arrival time, each difference being between shear wave arrival times at two adjacent points among the plurality of measurement points, and detect the occurrence of a reverberation by comparing a preset threshold with a value obtained by dividing the first average shear wave arrival time by the second average shear wave arrival time. In this case, the ultrasound diagnostic apparatus 300 may detect the occurrence of a reverberation when the first average shear wave arrival time is greater than a preset reference time.

According to an embodiment, the ultrasound diagnostic apparatus 300 may calculate a first shear wave velocity by dividing an average of distances of the plurality of measurement points by an average of shear wave arrival times at the plurality of measurement points, calculate a second shear wave velocity by dividing a distance between two adjacent points among the plurality of measurement points by a difference between shear wave arrival times respectively measured at the two adjacent measurement points , and detect the occurrence of a reverberation based on the first and second shear wave velocities. In this case, the ultrasound diagnostic apparatus 300 may divide a difference between the first and second shear wave velocities by the first shear wave velocity and detect the occurrence of a reverberation by comparing the resulting ratio with a preset threshold. For example, when the resulting ratio is greater than or equal to 0.5, the ultrasound diagnostic apparatus 300 may determine that the reverberation has occurred.

In operation S450, the ultrasound diagnostic apparatus 300 may display information about the detected reverberation on the display 330. According to an embodiment, the ultrasound diagnostic apparatus 300 may display information about a detected reverberation via a UI including at least one of a phrase, a sentence, a symbol, and a color. The ultrasound diagnostic apparatus 300 may display information about a detected reverberation, together with an ultrasound image of the object.

Although not illustrated as a separate operation in FIG. 4, the ultrasound diagnostic apparatus 300 may output information about the detected reverberation in the form of a sound including at least one of a beep sound, a melody, and a voice.

In general, during ultrasound elastography imaging, a reverberation occurs when an ultrasound signal transmitted to an object is reflected between surfaces of the probe 310 and a tissue or between tissues. A reverberation may appear as a relatively bright blurry band in an ultrasound image, e.g. a B-mode image. When elasticity of an object having a thick fat layer, such as an obese patient, is measured, a relatively severe reverberation may occur due to reflections between a surface of the probe and tissue or between tissues, or otherwise an elasticity value may be accurately measured due to a mild reverberation. When a reverberation occurs, the accuracy of an RMI tends to be degraded, and thus, the user is unable to obtain a reliable elasticity value.

According to the embodiments described with reference to FIGS. 3 and 4, the ultrasound diagnostic apparatus 300 may propagate a shear wave in an ROI of the object, measure shear wave arrival times at a plurality of measurement points, detect a reverberation based on the shear wave arrival times, and display information about the detected reverberation, thereby improving accuracy of elasticity measurement. Furthermore, the ultrasound diagnostic apparatus 300 may display information of reverberation together with an ultrasound image, such that, when a reverberation occurs, the user may manipulate the probe 310 to prevent a severe reverberation or may move an ROI to a region of mild reverberation, thereby facilitating elasticity measurement and increasing user convenience.

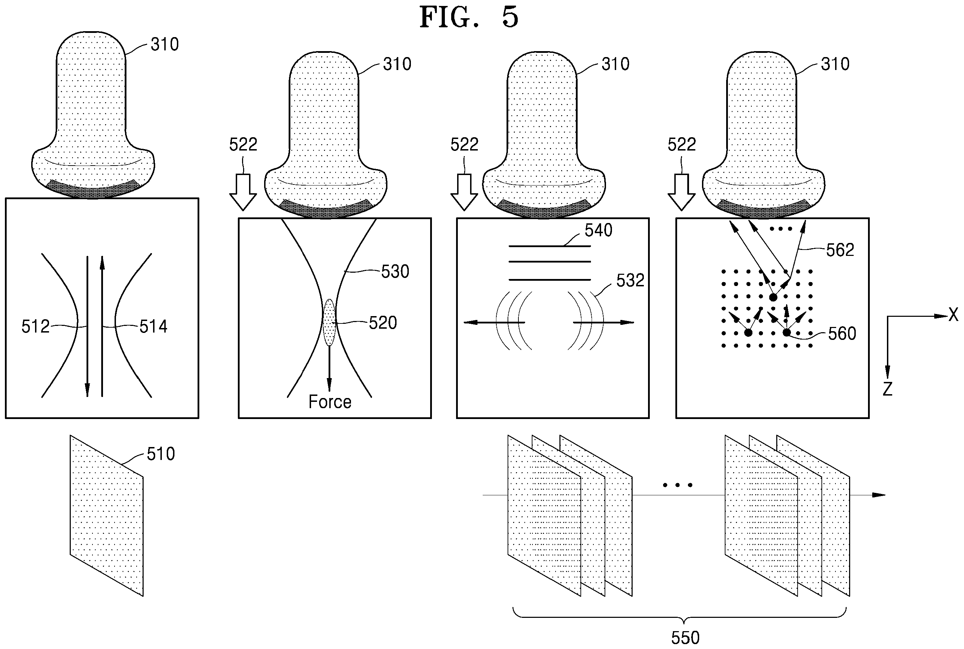

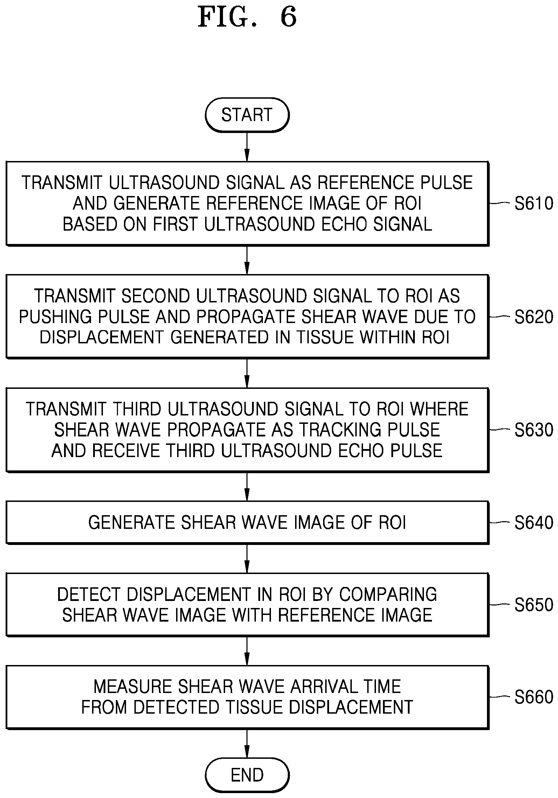

FIG. 5 is a diagram for explaining a method, performed by the ultrasound diagnostic apparatus 300, of inducing a displacement in a tissue within an ROI and calculating the displacement, according to an embodiment of the disclosure. FIG. 6 is a flowchart of a method, performed by the ultrasound diagnostic apparatus 300, of measuring a shear wave arrival time from a tissue displacement within an ROI, according to an embodiment of the disclosure. A method of operating the ultrasound diagnostic apparatus 300 will now be described in detail with reference to FIGS. 5 and 6.

Referring to FIGS. 5 and 6, the ultrasound diagnostic apparatus 300 transmits a first ultrasound signal 512 to an ROI of the object as a reference pulse and receives a first ultrasound echo signal 514 reflected in response to the first ultrasound signal 512. Furthermore, the ultrasound diagnostic apparatus 300 generates a reference ultrasound image 510 of the ROI based on the received first ultrasound echo signal 514 (operation S610).

According to an embodiment, as the ultrasound diagnostic apparatus 300 receives the first ultrasound echo signal 514, the processor (320 of FIG. 3) may generate the reference ultrasound image 510 of the ROI based on the first ultrasound echo signal 514. The reference ultrasound image 510 may be an image showing a position of tissue before a force is applied to the ROI. The reference ultrasound image 510 may be a B-mode or M-mode image of the ROI.

In operation S620, the ultrasound diagnostic apparatus 300 transmits via the probe 310 a second ultrasound signal 530 to a focal point 520 within the ROI as a pushing pulse and propagates a shear wave 532 due to a displacement generated in a tissue within the ROI. The second ultrasound signal 530 may be a focused ultrasound beam.

As the second ultrasound signal 530 is transmitted to the focal point 520 within the ROI, the shear wave 532 may be generated in the tissue located in the ROI. For example, a focused ultrasound beam transmitted to the ROI in a Z-axis direction may push a tissue in a direction of an ultrasound pulse (an axial direction), i.e., in an X-axis direction. Movement of the tissue located at the focal point 520 in the axial direction may cause an adjacent tissue to move in the X-axis (axial) direction. As the tissue adjacent to the focal point 520 moves in the same direction, the movement may be propagated sequentially to a tissue adjacent to the moving tissue. In this case, a force of an ultrasound pulse that moves the tissue may be referred to an acoustic force.

As movement is propagated to an adjacent tissue, an acoustic force applied to the focal point 520 may create a wave that propagates away from the focal point 520 as a point of an origin in a direction (a lateral direction) orthogonal to a direction of an ultrasound pulse. A wave propagating in a direction orthogonal to the direction of the ultrasound pulse may be referred to as the shear wave 532.

A propagation velocity of the shear wave 532 may be determined according to stiffness, Young's modulus, or shear modulus of tissue. For example, the propagation velocity of the shear wave 532 may vary from 1 to 10 m/s depending on the stiffness of tissue. Furthermore, the greater the stiffness of tissue, the higher the propagation velocity of the shear wave 532 in the tissue.

Furthermore, a relationship between the propagation velocity of the shear wave 532 through the tissue and the stiffness of tissue may be shown in an equation below.

$$G = \rho \times C^2$$

In this regard, G is tissue stiffness, $\rho$ is tissue density, and C is the propagation velocity of the shear wave 532. Tissue density $\rho$ may be considered as a constant value in the ROI and may be usually a known value. Accordingly, tissue stiffness indicating the rigidity of tissue may be detected as a quantitative value by measuring the propagation velocity of the shear wave 532 through the tissue.

The shear wave 532 may be detected by measuring a displacement of the tissue in the direction of an ultrasound pulse (an axial direction). The displacement of the tissue may be a distance by which the tissue moves in the axial direction with respect to the reference ultrasound image 510. Furthermore, the propagation velocity of the shear wave 532 through a sub-tissue in the ROI may be calculated based on a time point when displacements of the sub-tissue and tissue surrounding the sub-tissue arc maximum.

In operation S630, the ultrasound diagnostic apparatus 300 transmits, as a tracking pulse, a third ultrasound signal 540 to the ROI in which the shear wave 532 propagates and receives a third ultrasound echo pulse 562. Referring to FIG. 5, to detect a displacement of tissue generated by an acoustic force, the processor 320 may transmit the third ultrasound signal 540 to the ROI. In this case, to more accurately measure the propagation velocity of the shear wave 532, the processor 320 may transmit a plane wave to the ROI as the third ultrasound signal 540. When the plane wave is transmitted as the third ultrasound signal 540, the ultrasound diagnostic apparatus 300 may capture the shear wave 532 at a frame rate of several thousand fps.

After transmission to the tissue, the third ultrasound signal 540 may be scattered by a scatter 560 in a tissue within the ROI. The third ultrasound signal 540 scattered by the scatter 560 may be reflected to the probe 310. In this case, the third ultrasound signal 540 scattered by the scatter 560 may be referred to as the third ultrasound echo pulse 562.

In operation S640, the ultrasound diagnostic apparatus 300 generates a shear wave image 550 of the ROI based on the received third ultrasound echo pulse 562. As the third ultrasound echo pulse 562 is received, the processor 320 may generate an ultrasound image of the ROI. An image including a shear wave among ultrasound images generated based on the third ultrasound echo pulse 562 may be referred to as the shear wave image 550. When a plane wave is transmitted as the third ultrasound signal 540, the processor 320 may generate the shear wave image 550 at a frame rate of several thousand fps.

In operation S650, the ultrasound diagnostic apparatus 300 detects a tissue displacement in the ROI by comparing the shear wave image 550 with the reference ultrasound image 510. According to an embodiment, the processor 320 may respectively down shift the reference ultrasound image 510 and the shear wave image 550 to baseband and convert the result into demodulated data. In this case, the processor 320 may include a computation module for calculating a phase difference between the reference ultrasound image 510 and the shear wave image 550 based on the demodulated data and determining a displacement of tissue by converting the calculated phase difference into a distance by which tissue moves in the ROI. According to another embodiment, the processor 320 may interpolate the reference ultrasound image 510 and the shear wave image 550 and then detect a plurality of tissue displacements by calculating via cross-correlation a time delay in a scan line centered about a position of each axis.

In operation S660, the ultrasound diagnostic apparatus 300 measures a shear wave arrival time from the detected tissue displacement. According to an embodiment, the processor 320 may measure shear wave arrival times from displacements of a plurality of tissues respectively positioned at a plurality of measurement points that are at preset distances from the focal point 520 in the axial direction. In this case, the processor 320 may determine a time point when the magnitude of a change in each of a plurality of tissue displacements is maximum as a shear wave arrival time. To achieve this, the processor 320 may differentiate a plurality of detected tissue displacements with respect to time, respectively calculate axial velocities with respect to time for the differentiated tissue displacements, and respectively determine time points when the calculated axial velocities reach their maximum values as shear wave arrival times. However, a method of measuring a shear wave arrival time is not limited to the above-described method.

According to another embodiment, the processor 320 measure a shear wave arrival time by calculating, via cross-correlation, a time delay between a displacement signal according to the changes over time at a measurement point where the shear wave arrival time is to be measured and a displacement signal at another measurement point that is adjacent to the measurement point or a position where a shear wave is initially generated.

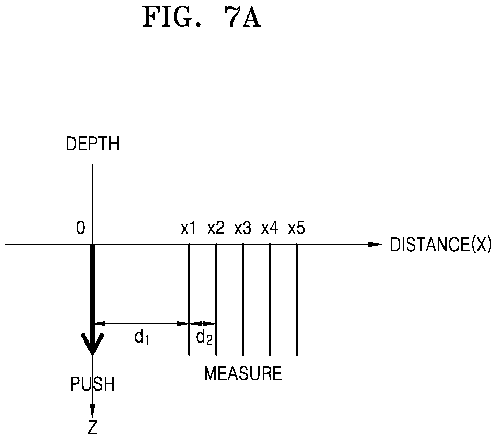

FIG. 7A is a graphical representation of coordinates for locations of a plurality of scan lines in an ROI and a focusing direction of a focused ultrasound beam applied to the ROI in a depth direction.

Referring to FIG. 7A, the probe 310 may transmit a focused ultrasound beam including a pushing pulse to an ROI of an object in a depth (Z-axis) direction for a preset time period. In this case, a shear wave may propagate in an axial (X-axis) direction due to a displacement caused by movement of tissue in the ROI. The shear wave propagates away from a focal point where a focused ultrasound beam is transmitted in both X-axis directions. However, for convenience of explanation, only the positive X-axis direction (+X direction) is shown in FIG. 7A while the negative X-axis direction (-X direction) is not shown.

A plurality of scan lines $x_1$, $x_2$, $x_3$, $x_4$, and $x_5$) are arranged in the ROI such that they are respectively spaced apart by preset distances from an ultrasound focal point O in an axial (X-axis) direction. The plurality of scan lines $x_1$, $x_2$, $x_3$, $x_4$, and $x_5$ may each extend in the depth (Z-axis) direction.

A first scan line $x_1$ may be spaced apart by a first distance $d_1$ from the ultrasound focal point O. For example, the first distance $d_1$ may be 5 mm. This is merely an example of a numerical value, and the first distance $d_1$ is not limited thereto. The plurality of scan lines $x_1$, $x_2$, $x_3$, $x_4$, and $x_5$ are spaced apart from one another by a second distance $d_2$. For example, the second distance $d_2$ that is the distance between the first and second scan lines $x_1$ and $x_2$ may be 1.44 mm. This is merely an example of a numerical value, and the second distance $d_2$ is not limited thereto.

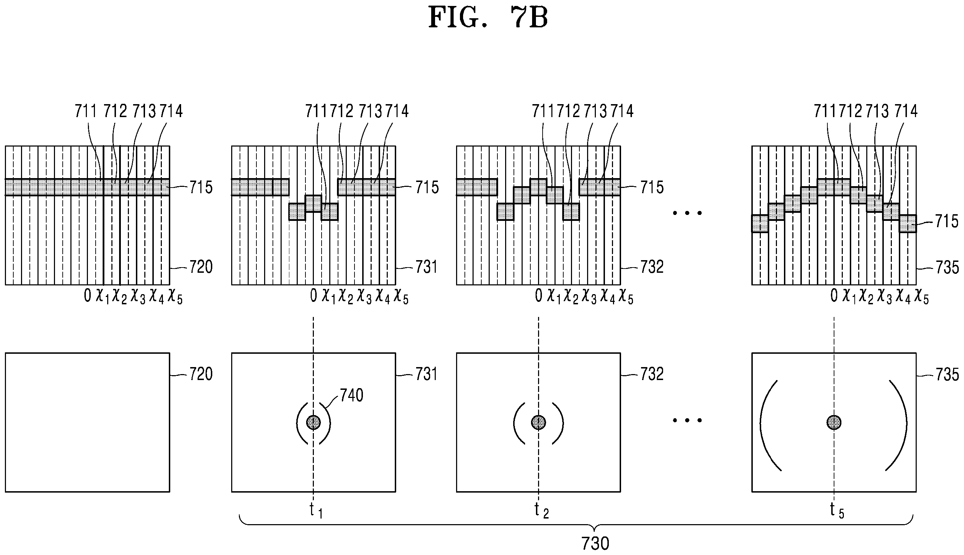

The plurality of scan lines $x_1$, $x_2$, $x_3$, $x_4$, and $x_5$ may respectively include a plurality of measurement points arranged at a specific depth value in the axial (X-axis) direction. According to an embodiment, the ultrasound diagnostic apparatus 300 may measure shear wave arrival times from displacements of a plurality of tissues respectively corresponding to the plurality of measurement points in the ROI. Measurement of a shear wave arrival time will now be described in detail with reference to FIG. 7B.

FIG. 7B is a diagram for explaining a method, performed by the ultrasound diagnostic apparatus 300, of calculating a shear wave propagation velocity at a plurality of measurement points within an ROI, according to an embodiment of the disclosure.

Referring to FIG. 7B, the processor 320 of the ultrasound diagnostic apparatus 300 may detect a displacement of an ROI based on a displacement of tissue generated by an acoustic force and measure a shear wave arrival time based on the detected displacement. A shear wave 740 may propagate in an axial direction based on a displacement of tissue. According to an embodiment, the processor 320 may compare shear wave images 730 with a reference ultrasound image 720 to calculate displacements of a plurality of sub-tissues 711 through 715 of the tissue within an ROI, which are respectively arranged at locations corresponding to a plurality of scan lines $x_1$, $x_2$, $x_3$, $x_4$, and $x_5$.

For example, the processor 320 may detect, based on cross-correlation, a position to which a first sub-tissue 711 in the reference ultrasound image 720 moves, in a first shear wave image 731. The first sub-tissue 711 may be a sub-tissue located in a region of the tissue within the ROI where the first scan line $x_1$ is arranged. The processor 320 may calculate a displacement of the first sub-tissue 711 in the axial direction via the first scan line $x_1$. The processor 320 may detect, based on the calculated displacement, a time point when the displacement of the first sub-tissue 711 is maximum. The processor 320 may determine the time point $t_1$ when the displacement of the first sub-tissue 711 is maximum as a time when the shear wave 740 arrives at the first sub-tissue 711. In this case, the processor 320 may determine the time point $t_1$ when the displacement of the first sub-tissue 711 is maximum as a shear wave arrival time $t_1$ for the first scan line $x_1$.

In the same manner as in the above-described method, the processor 320 may measure, in a plurality of shear wave images 731 through 735, time points $t_1$ through $t_5$ when shear waves respectively arrive at the plurality of sub-tissues 711 through 715 and determine the shear wave arrival times for the plurality of scan lines $x_1$ through $x_5$ based on the time points $t_1$ through $t_5$.

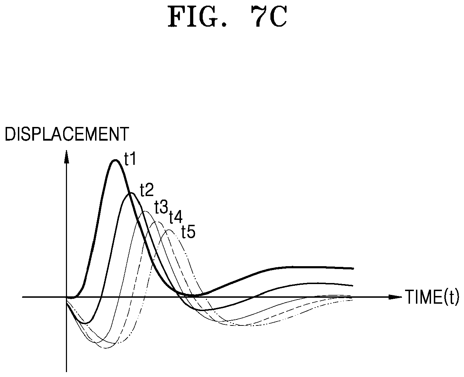

FIG. 7C is a graph illustrating a relationship between a shear wave arrival time and a tissue displacement measured by the ultrasound diagnostic apparatus 300 at each of a plurality of measurement points, according to an embodiment of the disclosure.

Referring to FIGS. 7B and 7C, a displacement of the first sub-tissue 711 reaches a maximum value at the time point $t_1$, and thus the time point $t_1$ is determined as a first shear wave arrival time that is a shear wave arrival time point for the first scan line $x_1$. Similarly, a displacement of a second sub-tissue 712 reaches a maximum value at the time point $t_2$, and thus the time point $t_2$ is determined as a second shear wave arrival time that is a shear wave arrival time point for the second scan line $x_2$.

Referring to FIGS. 7A through 7C, the processor 320 may calculate a velocity of the shear wave 740 based on the first distance $d_1$ by which each of the plu-

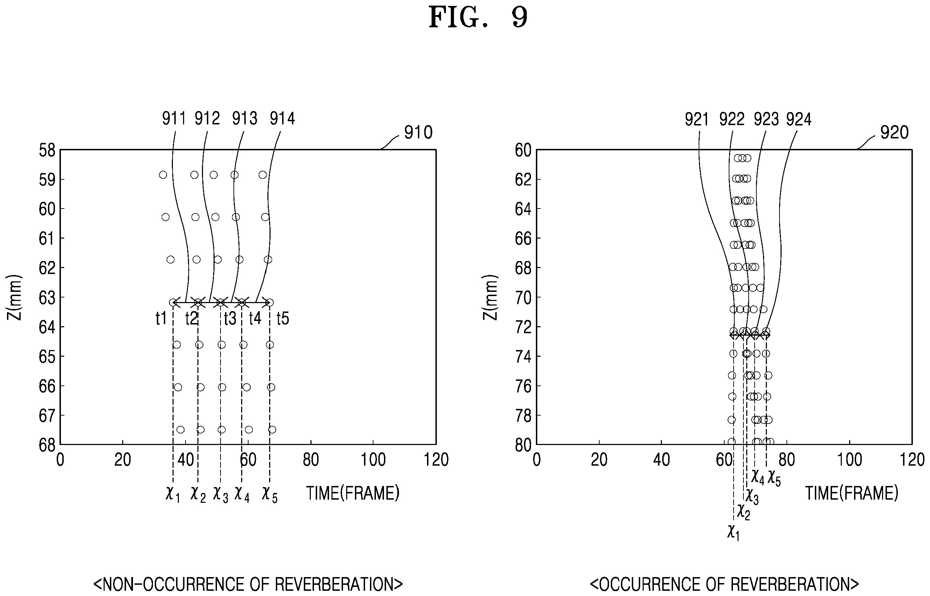

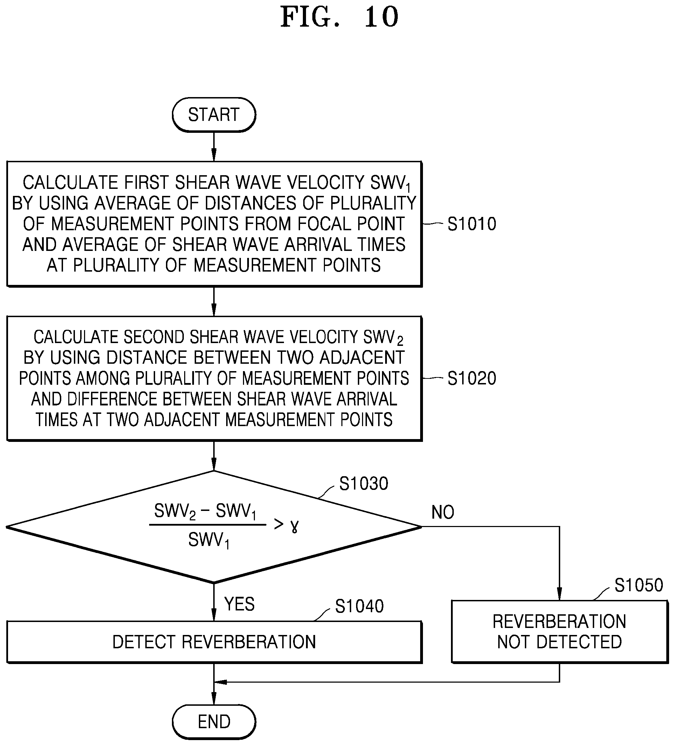

rality of scan lines $x_1$ through $x_5$ is spaced apart from the focal point O and the second distance $d_2$ that is the distance between adjacent ones of the plurality of scan lines $x_1$ through $x_5$. This will be described in detail below with reference to FIGS. 9 and 10.

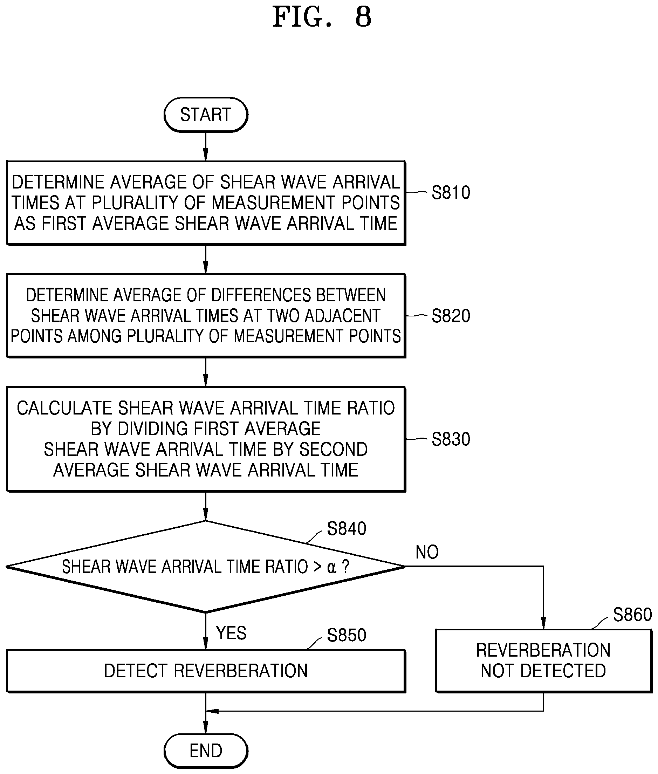

FIG. 8 is a flowchart of a method, performed by the ultrasound diagnostic apparatus 300, of detecting the occurrence of a reverberation based on shear wave arrival times at a plurality of measurement points within an ROI, according to an embodiment of the disclosure.

In operation S810, the ultrasound diagnostic apparatus 300 determines an average of shear wave arrival times respectively measured at a plurality of measurement points as a first average shear wave arrival $t_{avg1}$. According to an embodiment, the ultrasound diagnostic apparatus 300 may calculate the first average shear wave arrival time $t_{avg1}$ by adding all time points when displacements of sub-tissues respectively detected at a plurality of measurement points are maximum and then dividing the resulting sum by the number n of measurement points, according to an equation below.

$$t_{avg1} = \frac{\sum_{k=1}^{n} t_k}{n}$$

Referring to FIGS. 7A through 7C, the ultrasound diagnostic apparatus 300 may calculate the first average shear wave arrival time $t_{avg1}$ by adding all the time points, i. e., the first through fifth shear wave arrival times $t_1$ through $t_5$, when the displacements of sub-tissues respectively detected at the plurality of measurement points reach their maximum values, the measurement points separated in the axial direction at the same depth of the plurality of scan lines $x_1$ through $x_5$ along the depth direction, and dividing the resulting sum by 5 that is the number of measurement points.

In operation S820, the ultrasound diagnostic apparatus 300 determines an average of differences as a second average shear wave arrival time $t_{avg2}$, each difference being between shear wave arrival times at two adjacent points among the plurality of measurement points. According to an embodiment, the ultrasound diagnostic apparatus 300 may calculate the second average shear wave arrival time $t_{avg2}$ by adding all the differences between shear wave arrival times at two adjacent points among the plurality of measurement points and dividing the resulting sum by the number of pairs of two adjacent measurement points, i.e., n-1, according to an equation below.

$$t_{avg2} = \frac{\sum_{k=1}^{n-1} t_{k-1} - t_k}{n-1}$$

Referring to FIGS. 7A through 7C, the ultrasound diagnostic apparatus 300 may calculate the second average shear wave arrival time $t_{avg2}$ by performing an operation of adding all the differences between shear wave arrival times at two adjacent points among the plurality of measurement points, e.g., a difference $t_2 - t_1$ between the second and first shear wave arrival times $t_2$ and $t_1$, a difference $t_3 - t_2$ between the third and second shear wave arrival times $t_3$ and $t_2$, ..., and a difference $t_5 - t_4$ between the fifth and fourth shear wave arrival times $t_5$ and $t_4$ and then dividing the resulting sum by the number of pairs of two adjacent measurement points, i.e., 4 (5-1)

In operation S830, the ultrasound diagnostic apparatus 300 calculates a shear wave arrival time ratio $t_{ratio}$ by dividing the first shear wave arrival time $t_{avg1}$ by the second shear wave arrival time $t_{avg2}$. In an embodiment, the shear wave arrival time ratio $t_{ratio}$ may be calculated according to an equation below.

$$t_{ratio} = \frac{t_{avg1}}{t_{avg2}}$$

In operation S840, the ultrasound diagnostic apparatus 300 compares the shear wave arrival time ratio $t_{ratio}$ with a preset threshold $\alpha$. In this case, a value of the threshold $\alpha$ may be an arbitrary value set according to the type, specification, etc., of the ultrasound diagnostic apparatus 300. According to an embodiment, the value of the threshold $\alpha$ may be set based on a user input.

For example, the value of the threshold $\alpha$ may be 20. However, the value of the threshold $\alpha$ is not limited to the above values.

When the shear wave arrival time ratio $t_{ratio}$ is greater than the value of the threshold $\alpha$ in operation S840, the ultrasound diagnostic apparatus 300 detects a reverberation (operation S850). According to an embodiment, when it is determined that the shear wave arrival time ratio $t_{ratio}$ is greater than the value of the threshold, the ultrasound diagnostic apparatus 300 may determine that a reverberation has occurred due to a fat layer, etc., in an ROI.

When the shear wave arrival time ratio $t_{ratio}$ is less than the threshold $\alpha$, the ultrasound diagnostic apparatus 300 does not detect a reverberation (operation S860). According to an embodiment, in a case where the shear wave arrival time ratio $t_{ratio}$ is less than the threshold $\alpha$, the ultrasound diagnostic apparatus 300 may determine this case as an elastic environment in which a reverberation does not occur.

Although FIG. 8 illustrates an embodiment in which the ultrasound diagnostic apparatus 300 detects a reverberation according to a ratio between an average value $t_{avg1}$ of the shear wave arrival times at the plurality of measurement points and the average value $t_{avg2}$ of the differences between two adjacent points among the measurement points, embodiments of the disclosure are not limited thereto. According to another embodiment, when the first average shear wave arrival time $t_{avg1}$ is greater than a preset threshold $\beta$, the ultrasound diagnostic apparatus 300 may detect a reverberation. In this case, the threshold $\beta$ may be an arbitrary value that varies according to a frame rate of the shear wave images (730 of FIG. 7B).

Although not shown in FIG. 8, the ultrasound diagnostic apparatus 300 may detect a reverberation by comparing the first average shear wave arrival time $t_{avg1}$ with a minimum value of the differences between two adjacent points among the measurement points. According to another embodiment, the ultrasound diagnostic apparatus 300 may detect a reverberation by comparing the first average shear wave arrival time $t_{avg1}$ with a maximum value of the differences between two adjacent points among the measurement points. According to another embodiment, the ultrasound diagnostic apparatus 300 may detect a reverberation by comparing the shear wave arrival time $t_1$ at a measurement point that is closest to an ultrasound beam's focal point (the first scan line $x_1$ of FIG. 7A) with a preset threshold $\beta$.

FIG. 9 illustrates first and second wave front graphs 910 and 920 of shear wave arrival times respectively measured by the ultrasound diagnostic apparatus at a plurality of measurement points, according to an embodiment of the disclosure.

The first and second wave front graphs 910 and 920 of FIG. 9 respectively show shear wave arrival times with respect to a depth value of the scan lines $x_1$ through $x_5$ extending in a depth (Z-axis) direction and being separated from one another in an axial direction. The first and second wave front graphs 910 and 920 respectively show shear wave arrival times when a reverberation does not occur and when the reverberation occurs. The first and second wave front graphs 910 and 920 illustrate a case in which a shear wave image is captured at 6,250 fps. However, values respectively indicated on the first and second wave front graphs 910 and 920 are merely examples, and embodiments of the disclosure are not limited to the case in which a shear wave image is captured and obtained at 6,250 fps.

The first wave front graph 910 illustrates shear wave arrival times at measurement points when a depth value of the plurality of scan lines $x_1$ through $x_5$ is 63 mm. For example, first through fifth shear wave arrival times $t_1$ through $t_5$ may be about 5.44 ms, 6.72 ms, 8.32 ms, 9.28 ms, and 10.88 ms, respectively. The above values are all exemplary.

Referring to FIGS. 8 and 9, the ultrasound diagnostic apparatus 300 may calculate a first average shear wave arrival $t_{avg1}$ by adding all the first through fifth shear wave arrival times $t_1$ through $t_5$ in the first wave front graph 910 and dividing the resulting sum by 5 (operation S810). In this case, a value of the first average shear wave arrival time $t_{avg1}$ may be calculated as (5.44+6.72+8.32+9.28+10.88)/5 = 8.128 ms.

Furthermore, the ultrasound diagnostic apparatus 300 may determine a second average shear wave arrival time $t_{avg2}$ by calculating an average of differences between shear wave arrival times at two adjacent points among the measurement points (operation S820). The sum of a difference between the second and first shear wave arrival times $t_2$ and $t_1$ through a difference between the fifth and fourth shear wave arrival times $t_5$ and $t_4$ is 5.44 ms in the first wave front graph 910, and the second average shear wave arrival time $t_{avg2}$ may be calculated as 1. 36 ms by dividing 5.44 ms by 4.

In the first wave front graph 910, a shear wave arrival time ratio $t_{ratio}$ may be calculated as 5.976. The ultrasound diagnostic apparatus 300 may compare the shear wave arrival time ratio $t_{ratio}$ with the preset threshold $\alpha$ (operation S840). According to an embodiment, because a value of the threshold $\alpha$ is 20, the ultrasound diagnostic apparatus 300 does not detect a reverberation by using values from the first wave front graph 910 (operation S850).

The second wave front graph 920 illustrates shear wave arrival times at measurement points when a depth value of the plurality of scan lines $x_1$ through $x_5$ is 72 mm. It can be seen that shear wave arrival times in the second wave front graph 920 are arranged at relatively narrow intervals along the axial (X-axis) direction, as compared to those in the first wave front graph 910. For example, first through fifth shear wave arrival times $t_1$ through $t_5$ may be about 9.92 ms, 10.56 ms, 10.88 ms, 11.22 ms, and 11.84 ms, respectively. The above values are all exemplary.

Similarly, referring to FIGS. 8 and 9, the ultrasound diagnostic apparatus 300 may calculate a first average shear wave arrival $t_{avg1}$ by adding all the first through fifth shear wave arrival times $t_1$ through $t_5$ in the second wave front graph 920 and dividing the resulting sum by 5 (operation S810). In the second wave front graph 920, a value of the first average shear wave arrival time $t_{avg1}$ may be calculated as (9.92+10.56+10.88+11.22+11.84)/5 = 10.884 ms.

Furthermore, the ultrasound diagnostic apparatus 300 may determine a second average shear wave arrival time $t_{avg2}$ in the second wave front graph 920 (operation S820). The sum of a difference between the second and first shear wave arrival times $t_2$ and $t_1$ through a difference between the fifth and fourth shear wave arrival times $t_5$ and $t_4$ is 1.92 ms in the second wave front graph 920, and the second average shear wave arrival time $t_{avg2}$ may be calculated as 0.48 ms by dividing 1.92 ms by 4.

In the second wave front graph 920, a shear wave arrival time ratio $t_{ratio}$ may be calculated as 22.675. The ultrasound diagnostic apparatus 300 may compare the shear wave arrival time ratio $t_{ratio}$ with the preset threshold $\alpha$ (operation S840). According to an embodiment, because a value of the threshold $\alpha$ is 20, the ultrasound diagnostic apparatus 300 detects a reverberation by using values from the second wave front graph 920 (operation S850).

FIG. 10 is a flowchart of a method, performed by the ultrasound diagnostic apparatus 300, of detecting the occurrence of a reverberation based on a shear wave velocity calculated at a plurality of measurement points within an ROI, according to an embodiment of the disclosure.

In operation S1010, the ultrasound diagnostic apparatus 300 calculates a first shear wave velocity $swv_1$ by using an average of distances of a plurality of measurement points from a focal point and an average of shear wave arrival times respectively measured at the plurality of measurement points. According to an embodiment, the ultrasound diagnostic apparatus 300 may calculate the first shear wave velocity $swv_1$ by using an equation below.

$$swv_1 = \frac{\dfrac{\sum_{k=1}^{n} x_k}{n}}{\dfrac{\sum_{k=1}^{n} t_k}{n}}$$

Referring to FIGS. 7A and 10, a distance from the focal point O to the first scan line $x_1$ may be 5 mm. Furthermore, a distance between two adjacent scan lines may be 1.44 mm. However, the above values are exemplary. In this case, an average distance of the plurality of measurement points may be calculated as (5+6.44+7.88+9.32+10.76)/5=7.88 mm.

Referring to FIGS. 9 and 10, the first average shear wave arrival time is 8.128 ms in the first wave front graph 910, and thus, the first shear wave velocity $swv_1$ may be calculated as 0.969 m/s. However, the above values are exemplary. The second average shear wave arrival time $t_{avg2}$ is 10.884 ms in the second wave front graph 920, and thus, the first shear wave velocity $swv_1$ may be calculated as 0.72 m/s.

In operation S1020, the ultrasound diagnostic apparatus 300 calculates a second shear wave velocity $swv_2$ by using a distance between two adjacent points among the plurality of measurement points and a difference between shear wave arrival times respectively measured at the two adjacent measurement points. According to an embodiment, the ultrasound diagnostic apparatus 300 may calculate the second shear wave velocity $swv_2$ by using a distance between two arbitrary adjacent ones of the plurality of measurement points and a difference between shear wave arrival times, according to an equation below.

$$swv_2 = \frac{x_n - x_{n-1}}{t_n - t_{n-1}}$$

According to another embodiment, the ultrasound diagnostic apparatus 300 may calculate a plurality of shear wave velocities by using a distance between two adjacent points among the plurality of measurement points and a difference between shear wave arrival times and determine an average of the shear wave velocities as the second shear wave velocity $swv_2$.

Referring to FIGS. 9 and 10, a difference between the second and first shear wave arrival times $t_2$ and $t_1$ is 1.28 ms as seen in the first wave front graph 910, and thus, the second shear wave velocity $swv_2$.may be calculated as 1.44mm/1.28ms=1.125m/s. Similarly, a difference between the second and first shear wave arrival times $t_2$ and $t_1$ is 0.64 ms as seen in the second wave front graph 920, and thus, the second shear wave velocity $swv_2$ may be calculated as 1.44mm/0.64ms=2.25m/s. The above values are all exemplary.

In operation S1030, the ultrasound diagnostic apparatus 300 calculates a shear wave velocity ratio $swv_{ratio}$ and compares the shear wave velocity ratio $swv_{ratio}$ with a preset threshold $\gamma$. According to an embodiment, the ultrasound diagnostic apparatus 300 may calculate a shear wave velocity ratio $swv_{ratio}$ by dividing a difference between the second and first shear wave velocities $swv_2$ and $swv_1$ by the first shear wave velocity $swv_1$, according to an equation below.

$$swv_{ratio} = \frac{swv_2 - swv_1}{swv_1}$$

A value of the threshold $\gamma$ may be an arbitrary value set according to the type, specification, etc., of the ultrasound diagnostic apparatus 300. According to an embodiment, the value of the threshold $\gamma$ may be set based on a user input.

For example, the value of the threshold $\gamma$ may be 0.5. However, the value of the threshold $\gamma$ is not limited to the above values.

When the shear wave velocity ratio $swv_{ratio}$ is greater than the threshold $\gamma$ in operation S1030, the ultrasound diagnostic apparatus 300 detects a reverberation (operation S1040). According to an embodiment, when the shear wave velocity ratio $swv_{ratio}$ calculated in operation S1030 is greater than 0.5, the ultrasound diagnostic apparatus 300 may detect the occurrence of a reverberation.

Referring to FIGS. 9 and 10, in the first wave front graph 910, the shear wave velocity ratio $swv_{ratio}$ may be calculated as(1.125-0.969) / 0.969=0.16. Because the calculated shear wave velocity ratio $swv_{ratio}$ is less than 0.5 in the first wave front graph 910, the ultrasound diagnostic apparatus 300 does not detect a reverberation.

In the second wave front graph 920, the shear wave velocity ratio $swv_{ratio}$ may be calculated as 2.25-0.72)/0.72=2.125. Because the calculated shear wave velocity ratio $swv_{ratio}$ is greater than 0.5 in the second wave front graph 920, the ultrasound diagnostic apparatus 300 detects a reverberation.

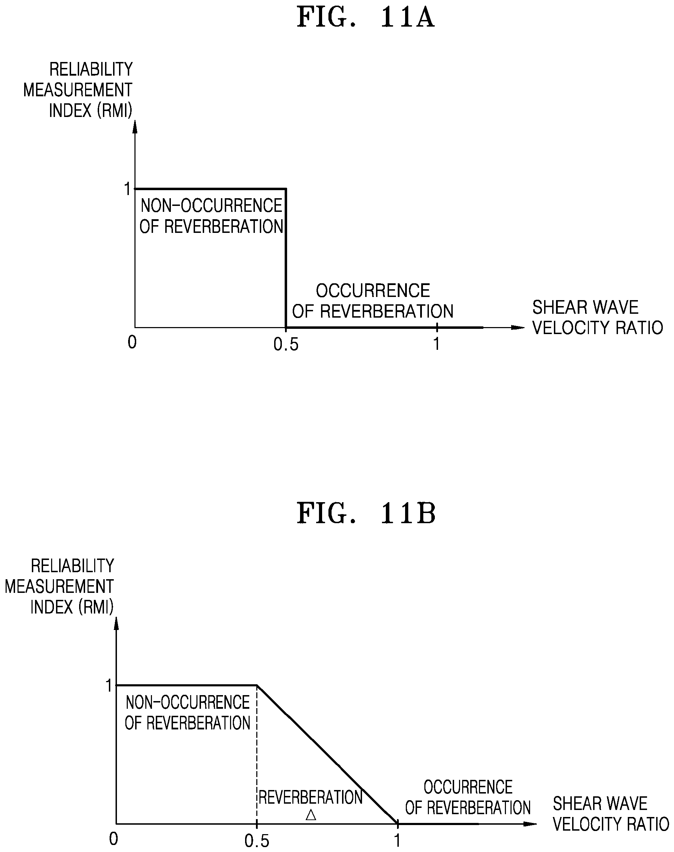

FIGS. 11A and 11B are graphs for explaining a method, performed by the ultrasound diagnostic apparatus 300, of determining a value of a RMI based on a shear wave velocity ratio, according to an embodiment of the disclosure.

Referring to FIG. 11A, the ultrasound diagnostic apparatus 300 may calculate a shear wave velocity ratio and determine a RMI based on the calculated shear wave velocity ratio. A RMI is an index value indicating the quality of a shear wave elastography image and may be replaced with a reliability index (RI) or cost function.

According to an embodiment, when a shear wave velocity ratio has a value that is greater than or equal to 0 but less than 0.5, the ultrasound diagnostic apparatus 300 may determine a value of RMI to be 1. In this case, the reliability is 100% which means that a reverberation does not occur. When the shear wave velocity ratio has a value that is greater than 0.5, the ultrasound diagnostic apparatus 300 may determine a value of RMI to be 0. In this case, the reliability is 0%, which means that a reverberation has occurred.

Referring to FIG. 11B, when a shear wave velocity ratio has a value that is greater than or equal to 0 but less than 0.5, the ultrasound diagnostic apparatus 300 may determine a value of RMI to be 1, like in FIG. 11A. However, when the shear wave velocity ratio has a value that is greater than or equal to 0.5 but less than 1, the ultrasound diagnostic apparatus 300 may determine a value of RMI according to an equation below.

$$RMI = -2 \times swv_{ratio} + 2$$

For example, when a calculated shear wave velocity ratio is 0.7, the ultrasound diagnostic apparatus 300 may determine a value of RMI to be 0.6. In this case, the reliability of shear wave elastography imaging may be expected to be 60%. Furthermore, the ultrasound diagnostic apparatus 300 may detect that a case in which the shear wave velocity ratio is 0.5 or more is an environment in which a reverberation has occurred.

When the shear wave velocity ratio is greater than or equal to 1, the ultrasound diagnostic apparatus 300 may determine a value of RMI to be 0.

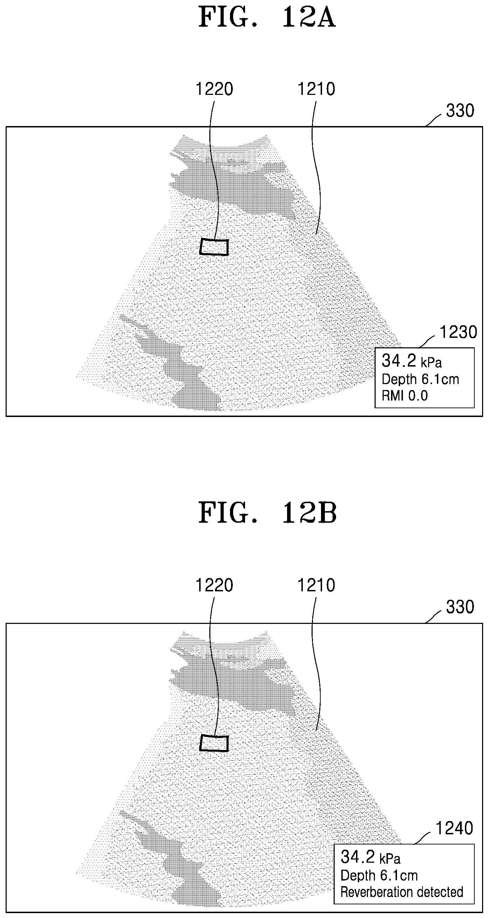

FIGS. 12A and 12B are diagrams for explaining a method, performed by the ultrasound diagnostic apparatus 300, of displaying information about a detected reverberation on the display 330, according to an embodiment of the disclosure.

Referring to FIG. 12A, the ultrasound diagnostic apparatus 300 may display on the display 330 an ROI interface 1220 and a reverberation information interface 1230, together with an ultrasound image 1210 of an object. According to an embodiment, the ultrasound image 1210 may be a B-mode image of the object. The ROI interface 1220 is displayed in the ultrasound image 1210 and is a UI indicating a position of an ROI set in the object.

The reverberation information interface 1230 may be displayed together with an interface for displaying an elasticity value and a depth value together with a RMI. According to an embodiment, the reverberation information interface 1230 may be displayed in a different color according to a value of RMI. For example, the reverberation information interface may be respectively shown in red, green, and blue colors when the value of RMI is 0, 0.5, and 1, respectively.

Although not shown in FIGS. 12A and 12B, according to an embodiment, the reverberation information interface 1230 may display a value of RMI as a percentage (%). For example, when the value of RMI is 0.6, the reverberation information interface 1230 may display the value of RMI as 60% after conversion into a percentage.

Referring to FIG. 12B, the ultrasound diagnostic apparatus 300 may display on the display 330 an ROI interface 1220 and a reverberation information interface 1240 , together with an ultrasound image 1210 of an object. The reverberation information interface 1240 may display reverberation information as at least one of a phrase, a sentence, a symbol. For example, when a reverberation is detected, the reverberation information interface 1240 may display reverberation information as a phrase or sentence such as "Reverb" or "Reverberation detected". Furthermore, when the reverberation is detected, the reverberation information interface 1240 may display a symbol such as "●".

According to an embodiment, the ultrasound diagnostic apparatus 300 may provide reverberation information via a UI in the form of a sound including at least one of a beep sound, a melody, and a voice. For example, the ultrasound diagnostic apparatus 300 may guide the user through a voice saying "Reverberation Detected" or notify the user of reverberation by making a beeping sound "beep-beep".

In the embodiments shown in FIGS. 12A and 12b, the ultrasound diagnostic apparatus 300 may display information about a detected reverberation together with the ultrasound image 1210 of the object, thereby allowing the user to more easily and conveniently detect the occurrence of the reverberation and thus improving user convenience.

The embodiments of the disclosure may be implemented as a software program including instructions stored in computer-readable storage media.

A computer may refer to a device capable of retrieving instructions stored in the computer-readable storage media and performing operations according to embodiments in response to the retrieved instructions, and may include ultrasound diagnostic apparatuses 300 according to the embodiments.

The computer-readable storage media may be provided in the form of non-transitory storage media. In this case, the term 'non-transitory' only means that the storage media do not include signals and are tangible, and the term does not distinguish between data that is semipermanently stored and data that is temporarily stored in the storage media.

In addition, the ultrasound diagnostic apparatuses 300 or methods according to embodiments may be included in a computer program product when provided. The computer program product may be traded, as a commodity, between a seller and a buyer.

The computer program product may include a software program and a computer-readable storage medium having stored thereon the software program. For example, the computer program product may include a product (e.g. a downloadable application) in the form of a software program electronically distributed by a manufacturer of an ultrasound diagnostic apparatus or through an electronic market (e.g., Google Play Store ™, and App Store ™). For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

In a system consisting of a server and a terminal (e.g., an ultrasound diagnostic apparatus), the computer program product may include a storage medium of the server or a storage medium of the terminal. Alternatively, in a case where a third device (e.g., a smartphone) is connected to the server or terminal through a communication network, the computer program product may include a storage medium of the third device. Alternatively, the computer program product may include a software program itself that is transmitted from the server to the terminal or the third device or that is transmitted from the third device to the terminal.

In this case, one of the server, the terminal, and the third device may execute the computer program product to perform methods according to embodiments of the disclosure. Alternatively, two or more of the server, the terminal, and the third device may execute the computer program product to perform the methods according to the embodiments in a distributed manner.

For example, the server (e.g., a cloud server, an artificial intelligence server, or the like) may run the computer program product stored therein to control the terminal communicating with the server to perform the methods according to the embodiments of the disclosure.

As another example, the third device may execute the computer program product to control the terminal communicating with the third device to perform the methods according to the embodiments. As a specific example, the third device may remotely control the ultrasound diagnostic apparatus 300 to transmit ultrasound signals to the object and generate an image of an inner area of the object based on information about signals reflected from the object.

As another example, the third device may execute the computer program product to directly perform the methods according to the embodiments based on a value received from an auxiliary device (e.g., a probe of a medical apparatus). As a specific example, the auxiliary device may transmit an ultrasound signal to an object and acquire an ultrasound signal reflected from the object. The third device may receive information about the reflected signal from the auxiliary device and generate an image of an inner area of the object based on the received information.

In a case where the third device executes the computer program product, the third device may download the computer program product from the server and execute the downloaded computer program product. Alternatively, the third device may execute the computer program product that is pre-loaded therein to perform the methods according to the embodiments of the disclosure.

Furthermore, while the embodiments of the disclosure have been illustrated and described above, the disclosure is not limited to the above-described specific embodiments, various modifications may be made therein by those of ordinary skill in the technical field to which the present disclosure pertains without departing from the gist of the disclosure that are claimed in the claims, and these modifications should not be understood individually from the perspective of the disclosure.

## Claims

1. A method of processing shear wave elastography data with respect to an object by using an ultrasound diagnostic apparatus (100, 300), the method comprising:

   inducing a shear wave in a region of interest of the object by emitting a focused ultrasound beam onto the region of interest of the object;
   obtaining ultrasound images of the object in which the shear wave is induced, respectively at a plurality of time points;
   measuring, by using the ultrasound images, shear wave arrival times respectively at a plurality of measurement points that are separated by preset distances from a focal point where the focused ultrasound beam is focused;
   detecting a reverberation in the region of interest based on the measured shear wave arrival times; and
   displaying information about the detected reverberation,
   **characterised in that**
   the detecting of the reverberation further compries;
   calculating a first shear wave velocity by dividing an average of distances of the plurality of measurement points by an average of the shear wave arrival times respectively measured at the plurality of measurement points;

   calculating a second shear wave velocity by dividing a distance between two adjacent points among the plurality of measurement points by a difference between shear wave arrival times respectively measured at the two adjacent measurement points; and
   detecting occurrence of the reverberation based on the first and second shear wave velocities;
   detecting the occurrence of the reverberation by comparing a difference between the second and first shear wave velocities with a preset threshold.

2. The method of claim 1, before the emitting of the focused ultrasound beam, further comprising transmitting a first ultrasound signal to the object and generating a reference ultrasound image by using an ultrasound echo signal reflected from the object, wherein the obtaining of the ultrasound images comprises transmitting a second ultrasound signal to the object in which the shear wave is induced and respectively obtaining a plurality of shear wave images at the plurality of time points by using an ultrasound echo signal reflected from the object.

3. The method of claim 2,
   wherein the measuring of the shear wave arrival times comprises: calculating a plurality of time points when displacements of tissues of the object respectively positioned at the plurality of measurement points respectively reach maximum values by comparing each of the plurality of shear wave images with the reference ultrasound image; and respectively determining the calculated plurality of time points as the shear wave arrival times at the plurality of measurement time points.

4. The method of claim 1, wherein the detecting of the reverberation comprises:
   determining an average of the shear wave arrival times respectively corresponding to the plurality of measurement points as a first average shear wave arrival time; determining an average of differences as a second average shear wave arrival time, each difference being between shear wave arrival times at two adjacent points among the plurality of measurement points; and detecting occurrence of the reverberation by comparing a value obtained by dividing the first average shear wave arrival time by the second average shear wave arrival time with a preset threshold.

5. An ultrasound diagnostic apparatus (100, 300) for processing shear wave elastography data with respect to an object, the ultrasound diagnostic apparatus comprising:

   an ultrasound probe (20, 310)

configured to induce a shear wave in a region of interest of the object by emitting a focused ultrasound beam onto the region of interest of the object;

a processor (320) configured to respectively obtain ultrasound images of the object at a plurality of time points, respectively measure shear wave arrival times at a plurality of measurement points that are separated by preset distances from a focal point where the focused ultrasound beam is focused, and detect a reverberation in the region of interest based on the measured shear wave arrival times; and

a display (140, 330) displaying information about the detected reverberation, **characterised in that** the processor (320) is further configured to calculate a first shear wave velocity by dividing an average of distances of the plurality of measurement points by an average of the shear wave arrival times respectively measured at the plurality of measurement points; and calculate a second shear wave velocity by dividing a distance between two adjacent points among the plurality of measurement points by a difference between shear wave arrival times respectively measured at the two adjacent measurement points; and detect occurrence of the reverberation based on the first and second shear wave velocities; and detect the occurrence of the reverberation by comparing a difference between the second and first shear wave velocities with a preset threshold.

6. The ultrasound diagnostic apparatus (100, 300) of claim 5,

   wherein the ultrasound probe (20, 310) is further configured to transmit a first ultrasound signal to the object before emitting the focused ultrasound beam, and the processor is further configured to generate a reference ultrasound image by using an ultrasound echo signal reflected from the object, and

   wherein the ultrasound images are a plurality of shear wave images respectively obtained by the processor at the plurality of time points.

7. The ultrasound diagnostic apparatus (100, 300) of claim 6,
   wherein the processor (320) is further configured to calculate a plurality of time points when displacements of tissues of the object respectively positioned at the plurality of measurement points respectively reach maximum values by comparing each of the plurality of shear wave images with the reference ultrasound image and to determine the calculated plurality of time points as the shear wave arrival times at the plurality of measurement time points.

8. The ultrasound diagnostic apparatus (100, 300) of claim 5,
   wherein the processor (320) is further configured to determine an average of the shear wave arrival times respectively corresponding to the plurality of measurement points as a first average shear wave arrival time, determine an average of differences as a second average shear wave arrival time, each difference being between shear wave arrival times at two adjacent points among the plurality of measurement points, and detect occurrence of the reverberation by comparing a value obtained by dividing the first average shear wave arrival time by the second average shear wave arrival time with a preset threshold.

9. The ultrasound diagnostic apparatus (100, 300) of claim 8,
   wherein the processor (320) is further configured to detect the occurrence of the reverberation by comparing the first average shear wave arrival time with a preset reference time.

10. The ultrasound diagnostic apparatus according to claim 5,

    wherein the processor (320) is further configured to determine a value obtained by dividing the difference between the second and first shear wave velocities by the first shear wave velocity as a reliability measurement index (RMI), and

    wherein the display (140, 330) further displays the RMI.

11. The ultrasound diagnostic apparatus of claim 5, wherein the display further displays the information about the detected reverberation via a user interface including at least one of a phrase, a sentence, a symbol, and a color.

12. A computer program product comprising instructions which, when the program is executed by an apparatus according to any one of claims 5 - 11, cause the apparatus to carry out the method of any one of claims 1 - 4.

**Patentansprüche**

1. Verfahren zum Verarbeiten von Scherwellen-Elastographiedaten in Bezug auf ein Objekt unter Verwendung einer Ultraschalldiagnosevorrichtung (100, 300), wobei das Verfahren Folgendes umfasst:

   Induzieren einer Scherwelle in einem Bereich von Interesse des Objekts durch Emittieren eines fokussierten Ultraschallstrahls auf den Bereich von Interesse des Objekts;

Erhalten von Ultraschallbildern des Objekts, in dem die Scherwelle induziert wird, jeweils zu einer Vielzahl von Zeitpunkten;

Messen, unter Verwendung der Ultraschallbilder, der jeweiligen Ankunftszeiten von Scherwellen an einer Vielzahl von Messpunkten, die durch voreingestellte Entfernungen von einem Fokuspunkt getrennt sind, an dem der fokussierte Ultraschallstrahl fokussiert wird;

Erfassen eines Nachhalls in dem Bereich von Interesse basierend auf den gemessenen Ankunftszeiten der Scherwellen; und

Anzeigen von Informationen über den erfassten Nachhall,

**dadurch gekennzeichnet, dass**

das Erfassen des Nachhalls ferner Folgendes umfasst:

Berechnen einer ersten Scherwellengeschwindigkeit durch Dividieren eines Durchschnitts der Entfernungen der Vielzahl von Messpunkten durch einen Durchschnitt der Ankunftszeiten der Scherwellen, die jeweils an der Vielzahl von Messpunkten gemessen wurden;

Berechnen einer zweiten Scherwellengeschwindigkeit durch Dividieren einer Entfernung zwischen zwei benachbarten Punkten unter der Vielzahl von Messpunkten durch eine Differenz zwischen Ankunftszeiten von Scherwellen, die jeweils an den zwei benachbarten Messpunkten gemessen wurden;

Erfassen des Auftretens des Nachhalls basierend auf der ersten und der zweiten Scherwellengeschwindigkeit; und

Erfassen des Auftretens des Nachhalls durch Vergleichen einer Differenz zwischen der zweiten und der ersten Scherwellengeschwindigkeit mit einem voreingestellten Schwellenwert.

2. Verfahren nach Anspruch 1, ferner umfassend, vor dem Emittieren des fokussierten Ultraschallstrahls, das Übertragen eines ersten Ultraschallsignals an das Objekt und Erzeugen eines Referenzultraschallbilds unter Verwendung eines von dem Objekt reflektierten Ultraschallechosignals, wobei das Erhalten der Ultraschallbilder das Übertragen eines zweiten Ultraschallsignals an das Objekt, in dem die Scherwelle induziert wird, und das jeweilige Erhalten einer Vielzahl von Scherwellenbildern zu der Vielzahl von Zeitpunkten unter Verwendung eines von dem Objekt reflektierten Ultraschallechosignals umfasst.

3. Verfahren nach Anspruch 2, wobei das Messen der Ankunftszeiten der Scherwellen Folgendes umfasst: Berechnen einer Vielzahl von Zeitpunkten, zu denen Verschiebungen von Geweben des Objekts, die jeweils an der Vielzahl von Messpunkten positioniert sind, jeweils Maximalwerte erreichen, und zwar durch Vergleichen jedes aus der Vielzahl von Scherwellenbildern mit dem Referenzultraschallbild; und jeweils Bestimmen der berechneten Vielzahl von Zeitpunkten als die Ankunftszeiten der Scherwellen an der Vielzahl von Messzeitpunkten.

4. Verfahren nach Anspruch 1, wobei das Erfassen des Nachhalls Folgendes umfasst: Bestimmen eines Durchschnitts der Ankunftszeiten der Scherwellen, die jeweils der Vielzahl von Messpunkten entsprechen, als eine erste durchschnittliche Ankunftszeiten von Scherwellen; Bestimmen eines Durchschnitts von Differenzen als eine zweite durchschnittliche Ankunftszeit von Scherwellen, wobei jede Differenz zwischen Ankunftszeiten von Scherwellen an zwei benachbarten Punkten aus der Vielzahl von Messpunkten besteht; und Erfassen des Auftretens des Nachhalls durch Vergleichen eines Werts, der durch Dividieren der ersten durchschnittlichen Ankunftszeit der Scherwelle durch die zweite durchschnittliche Ankunftszeit der Scherwelle erhalten wird, mit einem voreingestellten Schwellenwert.

5. Ultraschalldiagnosevorrichtung (100, 300) zum Verarbeiten von Scherwellen-Elastographiedaten in Bezug auf ein Objekt, wobei die Ultraschalldiagnosevorrichtung Folgendes umfasst:

eine Ultraschallsonde (20, 310), die dazu konfiguriert ist, eine Scherwelle in einem Bereich von Interesse des Objekts zu induzieren, indem sie einen fokussierten Ultraschallstrahl auf den Bereich von Interesse des Objekts emittiert;

einen Prozessor (320), der zu Folgendem konfiguriert ist: jeweiliges Erhalten von Ultraschallbildern des Objekts zu einer Vielzahl von Zeitpunkten, jeweiliges Messen der Ankunftszeiten von Scherwellen an einer Vielzahl von Messpunkten, die durch voreingestellte Entfernungen von einem Fokuspunkt getrennt sind, an dem der fokussierte Ultraschallstrahl fokussiert wird, und Erfassen eines Nachhalls in dem Bereich von Interesse basierend auf den gemessenen Ankunftszeiten der Scherwellen; und

eine Anzeige (140, 330), die Informationen über den erfassten Nachhall anzeigt,

**dadurch gekennzeichnet, dass** der Prozessor (320) ferner konfiguriert ist zum Berechnen einer ersten Scherwellengeschwindigkeit durch Dividieren eines Durchschnitts der Entfernungen der Vielzahl von Messpunkten durch einen Durchschnitt der Ankunftszeiten der Scherwellen, die jeweils an der Vielzahl von Messpunkten gemessen wurden; und Berechnen einer zweiten Scherwellengeschwindigkeit durch Dividieren einer Entfernung zwischen zwei benachbarten Punkten unter der Vielzahl von Messpunkten durch eine Differenz zwischen Ankunftszeiten von Scherwellen, die jeweils an den zwei benachbarten Messpunkten gemessen wurden; und Erfassen des Auftretens des Nachhalls basierend auf der ersten und der zweiten Scherwellengeschwindigkeit; und Erfassen des Auftretens des Nachhalls durch Vergleichen einer Differenz zwischen der zweiten und der ersten Scherwellengeschwindigkeit mit einem voreingestellten Schwellenwert.

6. Ultraschalldiagnosevorrichtung (100, 300) nach Anspruch 5, wobei die Ultraschallsonde (20, 310) ferner konfiguriert ist, um ein erstes Ultraschallsignal an das Objekt zu übertragen, bevor sie den fokussierten Ultraschallstrahl emittiert, und der Prozessor ferner konfiguriert ist, um ein ReferenzUltraschallbild unter Verwendung eines von dem Objekt reflektierten Ultraschallechosignals zu erzeugen, und
wobei es sich bei den Ultraschallbildern um eine Vielzahl von Scherwellenbildern handelt, die jeweils durch den Prozessor zu der Vielzahl von Zeitpunkten erhalten wurden.

7. Ultraschalldiagnosevorrichtung (100, 300) nach Anspruch 6, wobei der Prozessor (320) ferner konfiguriert ist zum Berechnen einer Vielzahl von Zeitpunkten, zu denen Verschiebungen von Geweben des Objekts, die jeweils an der Vielzahl von Messpunkten positioniert sind, jeweils Maximalwerte erreichen, und zwar durch Vergleichen jedes aus der Vielzahl von Scherwellenbildern mit dem Referenzultraschallbild, und zum Bestimmen der berechneten Vielzahl von Zeitpunkten als die Ankunftszeiten der Scherwellen an der Vielzahl von Messzeitpunkten.

8. Ultraschalldiagnosevorrichtung (100, 300) nach Anspruch 5, wobei der Prozessor (320) ferner zu Folgendem konfiguriert ist: Bestimmen eines Durchschnitts der Ankunftszeiten der Scherwellen, die jeweils der Vielzahl von Messpunkten entsprechen, als eine erste durchschnittliche Ankunftszeiten von Scherwellen, Bestimmen eines Durchschnitts von Differenzen als eine zweite durchschnittliche Ankunftszeit von Scherwellen, wobei jede Differenz zwischen Ankunftszeiten von Scherwellen an zwei benachbarten Punkten aus der Vielzahl von Messpunkten besteht, und Erfassen des Auftretens des Nachhalls durch Vergleichen eines Werts, der durch Dividieren der ersten durchschnittlichen Ankunftszeit der Scherwelle durch die zweite durchschnittliche Ankunftszeit der Scherwelle erhalten wird, mit einem voreingestellten Schwellenwert.

9. Ultraschalldiagnosevorrichtung (100, 300) nach Anspruch 8, wobei der Prozessor (320) ferner konfiguriert ist, um das Auftreten des Nachhalls durch Vergleichen der ersten durchschnittlichen Ankunftszeit der Scherwelle mit einer voreingestellten Referenzzeit zu erfassen.

10. Ultraschalldiagnosevorrichtung nach Anspruch 5, wobei der Prozessor (320) ferner konfiguriert ist, um einen Wert zu bestimmen, der durch Dividieren der Differenz zwischen der zweiten und der ersten Scherwellengeschwindigkeit durch die erste Scherwellengeschwindigkeit als Zuverlässigkeitsmessindex (RMI) erhalten wird, und
wobei die Anzeige (140, 330) ferner den RMI anzeigt.

11. Ultraschalldiagnosevorrichtung nach Anspruch 5, wobei die Anzeige ferner die Informationen über den erfassten Nachhall über eine Benutzerschnittstelle anzeigt, die mindestens eines von einer Phrase, einem Satz, einem Symbol und einer Farbe umfasst.

12. Computerprogrammprodukt, das Anweisungen umfasst, die, wenn das Programm von einer Vorrichtung nach einem der Ansprüche 5 bis 11 ausgeführt wird, die Vorrichtung veranlassen, das Verfahren nach einem der Ansprüche 1 bis 4 auszuführen.

**Revendications**

1. Procédé de traitement de données d'élastographie par ondes de cisaillement eu égard à un objet au moyen d'un appareil d'échographie diagnostique (100, 300), le procédé comprenant :

l'induction d'une onde de cisaillement dans une région d'intérêt de l'objet par émission d'un faisceau d'ultrasons focalisé sur la région d'intérêt de l'objet,
l'obtention d'images échographiques de l'objet dans lequel l'onde de cisaillement est induite, à une pluralité d'instants respectifs,
la mesure, à l'aide des images échographiques, des instants d'arrivée de l'onde de cisaillement respectifs au niveau d'une pluralité de points de mesure qui sont à des distances prédéfinies d'un point focal où est focalisé le faisceau d'ultrasons focalisé,
la détection d'une réverbération dans la région d'intérêt compte tenu des instants d'arrivée de l'onde de cisaillement mesurés, et
l'affichage d'informations concernant la réverbération détectée ;
**caractérisé en ce que**
la détection de la réverbération comprend en outre :

le calcul d'une première vitesse d'onde de cisaillement par division de la moyenne des distances de la pluralité de points de mesure par la moyenne des instants d'arrivée de l'onde de cisaillement respectivement mesurés au niveau de la pluralité de points de mesure,

le calcul d'une deuxième vitesse d'onde de cisaillement par division de la distance séparant deux points adjacents parmi la pluralité de points de mesure par une différence entre les instants d'arrivée de l'onde de cisaillement respectivement mesurés au niveau des deux points de points de mesure adjacents,

la détection de la survenue de la réverbération compte tenu des première et deuxième vitesses d'onde de cisaillement, et

la détection de la survenue de la réverbération par comparaison d'une différence entre les deuxième et première vitesses d'onde de cisaillement avec un seuil prédéfini.

2. Procédé selon la revendication 1, comprenant en outre, préalablement à l'émission du faisceau d'ultrasons focalisé, la transmission d'un premier signal ultrasonore à destination de l'objet et la génération d'une image échographique de référence à l'aide d'un signal d'écho ultrasonore réfléchi par l'objet, ladite obtention des images échographiques comprenant la transmission d'un deuxième signal ultrasonore à destination de l'objet dans lequel l'onde de cisaillement est induite et l'obtention respective d'une pluralité d'images d'onde de cisaillement à la pluralité d'instants à l'aide d'un signal d'écho ultrasonore réfléchi par l'objet.

3. Procédé selon la revendication 2, dans lequel la mesure des instants d'arrivée de l'onde de cisaillement comprend : le calcul d'une pluralité d'instants auxquels les déplacements des tissus de l'objet respectivement situés au niveau de la pluralité de points de mesure atteignent respectivement des valeurs maximales par comparaison de chacune des images de la pluralité d'images d'onde de cisaillement avec l'image échographique de référence ; et la détermination respective de la pluralité d'instants calculés comme étant les instants d'arrivée de l'onde de cisaillement à la pluralité d'instants de mesure.

4. Procédé selon la revendication 1, dans lequel la détection de la réverbération comprend : la détermination de la moyenne des instants d'arrivée de l'onde de cisaillement correspondant respectivement à la pluralité de points de mesure en tant que premier instant d'arrivé d'onde de cisaillement moyen ; la détermination de la moyenne des différences en tant que deuxième instant d'arrivé d'onde de cisaillement moyen, chacune des différences étant entre des instants d'arrivée de l'onde de cisaillement au niveau de deux points adjacents parmi la pluralité de points de mesure ; et la détection de la survenue de la réverbération par comparaison d'une valeur, obtenue par division du premier instant d'arrivé d'onde de cisaillement moyen par le deuxième instant d'arrivé d'onde de cisaillement moyen, avec un seuil prédéfini.

5. Appareil d'échographie diagnostique (100, 300) permettant de traiter des données d'élastographie par ondes de cisaillement eu égard à un objet, ledit appareil d'échographie diagnostique comprenant :

une sonde ultrasonore (20, 310) conçue pour induire une onde de cisaillement dans une région d'intérêt de l'objet par émission d'un faisceau d'ultrasons focalisé sur la région d'intérêt de l'objet,

un processeur (320) conçu pour obtenir des images échographiques de l'objet à une pluralité d'instants respectifs, pour mesurer les instants d'arrivée respectifs de l'onde de cisaillement au niveau d'une pluralité de points de mesure qui sont à des distances prédéfinies d'un point focal où est focalisé le faisceau d'ultrasons focalisé, et pour détecter une réverbération dans la région d'intérêt compte tenu des instants d'arrivée de l'onde de cisaillement mesurés, et

un écran (140, 330) affichant des informations concernant la réverbération détectée ;

**caractérisé en ce que** le processeur (320) est conçu en outre pour calculer une première vitesse d'onde de cisaillement par division de la moyenne des distances de la pluralité de points de mesure par la moyenne des instants d'arrivée de l'onde de cisaillement respectivement mesurés au niveau de la pluralité de points de mesure, pour calculer une deuxième vitesse d'onde de cisaillement par division de la distance séparant deux points adjacents parmi la pluralité de points de mesure par une différence entre les instants d'arrivée de l'onde de cisaillement respectivement mesurés au niveau des deux points de points de mesure adjacents, et pour détecter la survenue de la réverbération compte tenu des première et deuxième vitesses d'onde de cisaillement, et détecter la survenue de la réverbération par comparaison d'une différence entre les deuxième et première vitesses d'onde de cisaillement avec un seuil prédéfini.

6. Appareil d'échographie diagnostique (100, 300) selon la revendication 5, dans lequel la sonde ultrasonore (20, 310) est conçue en outre pour transmettre un premier signal ultrasonore à destination de l'objet, avant d'émettre le faisceau d'ultrasons focalisé, et le processeur est conçu en outre pour générer une image échographique de référence à l'aide d'un signal d'écho ultrasonore réfléchi par l'objet, et

dans lequel les images échographiques consistent en une pluralité d'images d'onde de cisaillement respectivement obtenues par le processeur à ladite pluralité d'instants.

7. Appareil d'échographie diagnostique (100, 300) selon la revendication 6, dans lequel le processeur (320) est conçu en outre pour calculer une pluralité d'instants auxquels les déplacements des tissus de l'objet respectivement situés au niveau de la pluralité de points de mesure atteignent respectivement des valeurs maximales par comparaison de chacune des images de la pluralité d'images d'onde de cisaillement avec l'image échographique de référence et pour déterminer la pluralité d'instants calculés comme étant les instants d'arrivée de l'onde de cisaillement à la pluralité d'instants de mesure.

8. Appareil d'échographie diagnostique (100, 300) selon la revendication 5, dans lequel le processeur (320) est conçu en outre pour déterminer la moyenne des instants d'arrivée de l'onde de cisaillement correspondant respectivement à la pluralité de points de mesure en tant que premier instant d'arrivé d'onde de cisaillement moyen, pour déterminer la moyenne des différences en tant que deuxième instant d'arrivé d'onde de cisaillement moyen, chacune des différences étant entre des instants d'arrivée de l'onde de cisaillement au niveau de deux points adjacents parmi la pluralité de points de mesure, et pour détecter la survenue de la réverbération par comparaison d'une valeur, obtenue par division du premier instant d'arrivé d'onde de cisaillement moyen par le deuxième instant d'arrivé d'onde de cisaillement moyen, avec un seuil prédéfini.

9. Appareil d'échographie diagnostique (100, 300) selon la revendication 8, dans lequel le processeur (320) est conçu en outre pour détecter la survenue de la réverbération par comparaison du premier instant d'arrivé d'onde de cisaillement moyen avec un instant de référence prédéfini.

10. Appareil d'échographie diagnostique selon la revendication 5, dans lequel le processeur (320) est conçu en outre pour déterminer une valeur obtenue par division de la différence entre les deuxième et première vitesses d'onde de cisaillement par la première vitesse d'onde de cisaillement en tant qu'indice de fiabilité de mesure (reliability measurement index, RMI), et

dans lequel l'écran (140, 330) affiche en outre ledit RMI.

11. Appareil d'échographie diagnostique selon la revendication 5, dans lequel l'écran affiche en outre les informations concernant la réverbération détectée, par le biais d'une interface utilisateur comportant une expression, une phrase, un symbole et/ou une couleur.

12. Produit-programme d'ordinateur comprenant des instructions qui, lorsque le programme est exécuté par un appareil selon l'une quelconque des revendications 5 à 11, amènent l'appareil à mettre en oeuvre le procédé selon l'une quelconque des revendications 1 à 4.

# FIG. 1

# FIG. 2A

# FIG. 2B

# FIG. 2C

200c

224

223

20

# FIG. 3

# FIG. 4

```
                         ┌─────────┐
                         │  START  │
                         └─────────┘
                              │
                              ▼
    ┌───────────────────────────────────────────────┐
    │      INDUCE SHEAR WAVE IN OBJECT BY EMITTING    │─── S410
    │    FOCUSED ULTRASOUND BEAM ONTO ROI OF OBJECT   │
    └───────────────────────────────────────────────┘
                              │
                              ▼
    ┌───────────────────────────────────────────────┐
    │        OBTAIN ULTRASOUND IMAGES OF OBJECT       │─── S420
    │           AT PLURALITY OF TIME POINTS           │
    └───────────────────────────────────────────────┘
                              │
                              ▼
    ┌───────────────────────────────────────────────┐
    │    MEASURE SHEAR WAVE ARRIVAL TIMES RESPECTIVELY│─── S430
    │      AT PLURALITY OF MEASUREMENT POINTS IN ROI  │
    └───────────────────────────────────────────────┘
                              │
                              ▼
    ┌───────────────────────────────────────────────┐
    │           DETECT REVERBERATION BASED ON         │─── S440
    │        MEASURED SHEAR WAVE ARRIVAL TIMES        │
    └───────────────────────────────────────────────┘
                              │
                              ▼
    ┌───────────────────────────────────────────────┐
    │  DISPLAY INFORMATION ABOUT DETECTED REVERBERATION│─── S450
    └───────────────────────────────────────────────┘
                              │
                              ▼
                         ┌─────────┐
                         │   END   │
                         └─────────┘
```

# FIG. 5

EP 3 603 527 B1

# FIG. 6

START

TRANSMIT ULTRASOUND SIGNAL AS REFERENCE PULSE AND GENERATE REFERENCE IMAGE OF ROI BASED ON FIRST ULTRASOUND ECHO SIGNAL — S610

TRANSMIT SECOND ULTRASOUND SIGNAL TO ROI AS PUSHING PULSE AND PROPAGATE SHEAR WAVE DUE TO DISPLACEMENT GENERATED IN TISSUE WITHIN ROI — S620

TRANSMIT THIRD ULTRASOUND SIGNAL TO ROI WHERE SHEAR WAVE PROPAGATE AS TRACKING PULSE AND RECEIVE THIRD ULTRASOUND ECHO PULSE — S630

GENERATE SHEAR WAVE IMAGE OF ROI — S640

DETECT DISPLACEMENT IN ROI BY COMPARING SHEAR WAVE IMAGE WITH REFERENCE IMAGE — S650

MEASURE SHEAR WAVE ARRIVAL TIME FROM DETECTED TISSUE DISPLACEMENT — S660

END

# FIG. 7A

# FIG. 7B

EP 3 603 527 B1

FIG. 7C

DISPLACEMENT

t1
t2
t3
t4
t5

TIME(t)

# FIG. 8

START

DETERMINE AVERAGE OF SHEAR WAVE ARRIVAL
TIMES AT PLURALITY OF MEASUREMENT POINTS
AS FIRST AVERAGE SHEAR WAVE ARRIVAL TIME — S810

DETERMINE AVERAGE OF DIFFERENCES BETWEEN
SHEAR WAVE ARRIVAL TIMES AT TWO ADJACENT
POINTS AMONG PLURALITY OF MEASUREMENT POINTS — S820

CALCULATE SHEAR WAVE ARRIVAL TIME RATIO
BY DIVIDING FIRST AVERAGE
SHEAR WAVE ARRIVAL TIME BY SECOND
AVERAGE SHEAR WAVE ARRIVAL TIME — S830

S840

SHEAR WAVE ARRIVAL TIME RATIO > α ?

NO

YES

S850

DETECT REVERBERATION

S860

REVERBERATION
NOT DETECTED

END

# FIG. 9

<NON-OCCURRENCE OF REVERBERATION>

<OCCURRENCE OF REVERBERATION>

EP 3 603 527 B1

# FIG. 10

START

CALCULATE FIRST SHEAR WAVE VELOCITY $SWV_1$ BY USING AVERAGE OF DISTANCES OF PLURALITY OF MEASUREMENT POINTS FROM FOCAL POINT AND AVERAGE OF SHEAR WAVE ARRIVAL TIMES AT PLURALITY OF MEASUREMENT POINTS — S1010

CALCULATE SECOND SHEAR WAVE VELOCITY $SWV_2$ BY USING DISTANCE BETWEEN TWO ADJACENT POINTS AMONG PLURALITY OF MEASUREMENT POINTS AND DIFFERENCE BETWEEN SHEAR WAVE ARRIVAL TIMES AT TWO ADJACENT MEASUREMENT POINTS — S1020

S1030

$$\frac{SWV_2 - SWV_1}{SWV_1} > \gamma$$

NO

YES — S1040

DETECT REVERBERATION

S1050

REVERBERATION NOT DETECTED

END

## FIG. 11A

RELIABILITY
MEASUREMENT
INDEX (RMI)

1

NON-OCCURRENCE
OF REVERBERATION

OCCURRENCE
OF REVERBERATION

SHEAR WAVE
VELOCITY RATIO

0        0.5        1

## FIG. 11B

RELIABILITY
MEASUREMENT
INDEX (RMI)

1

NON-OCCURRENCE
OF REVERBERATION

REVERBERATION
△

OCCURRENCE
OF REVERBERATION

SHEAR WAVE
VELOCITY RATIO

0        0.5        1

## FIG. 12A

1220   1210

330

1230

34.2 kPa
Depth 6.1cm
RMI 0.0

## FIG. 12B

1220   1210

330

1240

34.2 kPa
Depth 6.1cm
Reverberation detected

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Sonic Estimation of Elasticity via Resonance: A New Method of Assessing Homostasis. **COREY et al.** Annals of Biomedical Engineering. Springer, 2015, vol. 44